# EUROPEAN PATENT SPECIFICATION

(11) **EP 1 355 903 B1**
(45) Date of publication and mention of the grant of the patent: **16.03.2005**
(21) Application number: 02719740.9
(22) Date of filing: 30.01.2002
(51) Int. Cl.: C07D 401/14, A61K 31/444, A61P 35/00

(54) **PYRAZOLE DERIVATIVES AGAINST TGF OVEREXPRESSION**
PYRAZOLDERIVATE GEGEN TGF ÜBEREXPRIMIERUNG
DERIVES PYRAZOLE CONTRE LA SUREXPRESSION DU FACTEUR TGF

(30) Priority: 02.02.2001 GB 0102661; 09.08.2001 GB 0119424
(43) Date of publication of application: 29.10.2003
(73) Proprietor: SmithKline Beecham Corporation, Philadelphia, PA 19101 (US)
(72) Inventor: GELLIBERT, Francoise Jeanne, Lab. GlaxoSmithkline, F-91940 Les Ulis (FR); MATHEWS, Neil, GlaxoSmithKline, Stevenage, Hertfordshire SG1 2NY (GB)
(74) Representative: Sewell, Richard Charles
(86) International application number: PCT/EP2002/000938
(87) International publication number: WO 2002/066462

(56) References cited:
- WO-A-00/12497
- WO-A-01/72737
- WO-A-98/52937

## Description

The present invention relates to novel pyrazole derivatives, processes for the preparation thereof, the use thereof in therapy, particularly in the treatment or prophylaxis of disorders characterised by overexpression of transforming growth factor β (TGF-β), and pharmaceutical compositions for use in such therapy.

TGF-β is a multi-functional cytokine which belongs to the TGF-β superfamily which includes activins/inhibins, bone morphogenetic proteins (BMPs) and TGF-βs. Three isoforms of TGF-β (TGF-β1, TGF-β2, and TGF-β3) have been identified in mammals, each of which is encoded by a distinct gene on different chromosomes (D.A. Lawrence, *Eur. Cytokine. Netw.,* 1996, **7(3)**, 363). TGF-β initiates an intracellular signalling pathway which ultimately leads to the expression of genes that regulate cell cycle, control proliferative responses, or relate to extracellular matrix proteins that mediate cell adhesion, migration and intercellular communication. TGF-β has pleitropic effects including modulation of cell growth and differentiation, extracellular matrix formation, hematopoiesis, and immunomodulation (Roberts and Spoon, *Handbook of Experimental Pharmacology,* 1990, **95**, 419-458).

A variety of cell surface proteins and receptors are known to transduce the signals initiated by the binding of the active TGF-β ligand to its receptors. Initiation of the TGF-β signalling pathway results from the binding of the TGF-β ligand to the extracellular domain of the type II membrane receptor (Massague, *Ann. Rev. Biochem.,* 1998, **67**, 753.). The bound type II receptor then recruits type I (Alk5) receptor into a multimeric membrane complex, whereupon active type II receptor kinase phoshorylates and activates type I receptor kinase. The function of the type I receptor kinase is to phosphorylate a receptor-associated co-transcription factor, Smad-2 or Smad-3; thereby releasing it into the cytoplasm where it binds to Smad-4. The PAI-1 gene is activated by TGF-β as a consequence of the abovementioned cellular pathway.

One approach to the treatment and/or prophylaxis of disorders characterised by the overexpression of TGF-β is inhibition of the TGF-β signal transduction. For example, inhibition of the TGF-β type II receptor by overexpression of a dominant negative TGF-β type II receptor has previously been shown to prevent liver fibrosis and dysfunction in rat models (*Proc. Natl. Acad. Sci,* 1999, **96(5)**, 2345), and also to prevent progression of established liver fibrosis (*Hepatology,* 2000, **32**, 247).

Pathological overexpression of TGF-β is known to be associated with a number of undesirable effects, leading ultimately to the development of serious pathogenic conditions (G.C. Blobe *et al., N. Engl. J. Med.,* 2000, 1350). In particular, pathological overexpression of TGF-β may cause excessive accumulation of extracellular matrix (ECM), inhibition of cell proliferation and immunosupression. Excessive accumulation of ECM is known to lead to fibrotic diseases such as tumor fibrosis, radiation-induced fibrosis, fibrosis of the liver, kidney, lung, bowel, heart, pancreas, peritoneum or other organs. Fibrosis can lead to pathologic conditions such as cirrhosis, idiopathic pulmonary fibrosis, glomerulosclerosis and hypertrophic scars.

A number of other disease states are known to be associated with variations in the expression of genes which are controlled by TGF-β including cancer development, abnormal bone function and inflammatory disorders.

The development of compounds capable of inhibiting the TGF-β intracellular pathway is seen as a desirable way to effect prophylaxis and/or treatment of the above-mentioned conditions. Compounds capable of inhibiting the TGF-β intracellular pathway and/or the expression of TGF-β may be used in the treatment of disorders the symptoms of which often lead to the development of fibrotic conditions. For example, compounds of the present invention may be useful in treating the fibrosis associated with various liver-related conditions such as hepatitis B virus (HBV), hepatitis C virus (HCV), alcohol-induced hepatitis, haemochromatosis and primary biliary cirrhosis.

The compounds of the present invention are pyrazole derivatives. Other pyrazole compounds have previously been described for use in alternative medicinal applications. PCT Patent Applications, WO 96/03385, WO 98152937, WO 98/52940, WO 98152941 and WO 00/31063 (Searle & Co) disclose a series of substituted pyrazole compounds and their use in the treatment of p38 kinase mediated disorders. In particular the compounds described are useful in the treatment of inflammation, inflammatory bowel disease, multiple sclerosis and asthma. European Patent Application No. 0 846 687 (Lilly & Co) describes novel substituted pyrazoles useful for the inhibition of sPLA₂ mediated release of fatty acids. Such compounds are useful in the treatment of conditions such as septic shock. EP 0 846 686 (Pfizer Ltd) discloses a series of condensed pyrazole derivatives which act as inhibitors of both Interteukin-1 (IL-1) and tumor necrosis factor (TNF). Such compounds are useful in the treatment of IL-1 and TNF mediated diseases such as chronic inflammatory diseases, specific autoimmune disease and sepsis-induced organ injury. None of the aforementioned patent applications describe the pyrazole compounds of the present invention.

PCT Patent Application WO 00/12947 (Scios Inc.) describes the use of a series of quinazoline derivatives for treating various disorders associated with enhanced activity of kinase p38-α and/or TGF-β. The compounds described therein have been shown to inhibit the activities of both proteins and are therefore particularly useful for the treatment of conditions in which an enhanced activity towards both p38-α and TGF-β is required.

It has now been discovered that certain substituted pyrazole compounds, as described below, are useful in the treatment or prophylaxis of disorders characterised by the overexpression of TGF-β. In particular, compounds of the present invention are TGF-β inhibitors which act at the TGF-β type I (Alk5) receptor level.

According to one aspect of the present invention, we provide compounds of formula (I), 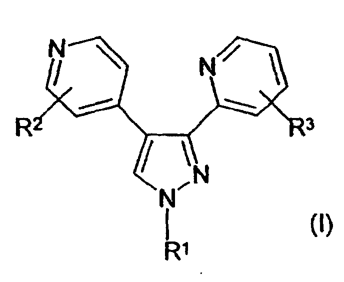 wherein,
R¹ is selected from H, C₁₋₄ alkyl or CH₂CONR⁴R⁵, wherein R⁴ is selected from H or C₁₋₄ alkyl and R⁵ is C₁₋₄ alkyl;
R² is selected from phenyl, furanyl or thiophenyl, wherein the phenyl may be further substituted by one or more substituents, which may be the same or different, selected from halo (such as fluoro, chloro, bromo), -CN, -CF₃, -OCF₃, C₁₋₄ alkyl, -OR⁶, -O-(CH₂)ₙ-XR⁶R⁷, -O-(CH₂)ₙ-OR⁶, -O-(CH₂)ₙ-COR⁶, -O-(CH₂)ₙ-C₂₋₆ alkenyl, -O-(CH₂)ₙ-C₂₋₆ alkynyl, -(CH₂)ₙ-NR⁶R⁷, -CONR⁶R⁷, -NHCOR⁶ and -NR⁶R⁷ where n is an integer value from 1 to 6 and where X is an atom selected from C, N or S, and wherein the furanyl and thiophenyl may be further substituted by one or more substituents, which may be the same or different, selected from halo (such as fluoro, chloro, bromb), -CN, -CF₃, -OH, -OCF₃, C₁₋₄ alkyl and C₁₋₄ alkoxy;
R³ is selected from H, halo (such as fluoro, chloro, bromo), -CN, -CF₃, C₁₋₄ alkyl or C₁₋₄ alkoxy;
R⁶ and R⁷ which may be the same or different, are selected from H, C₁₋₆ alkyl, cycloalkyl, aryl, arylC₁₋₆ alkyl, heteroaryl, heteroarylC₁₋₆ alkyl, heterocyclyl, heterocyclylC₁₋₆ alkyl, hydroxyC₁₋₆ alkyl, -(CH₂)ₙ-NR⁸R⁹, or R⁶ and R⁷ together with the atom to which they are attached form a 3, 4, 5, 6 or 7 membered saturated or unsaturated ring which may contain one or more heteroatoms selected from N, S or O, and wherein the ring may be further substituted by one or more substituents selected from halo (such as fluoro, chloro, bromo), -CN, -CF₃, -OH, -OCF₃, C₁₋₄ alkyl and C₁₋₄ alkoxy;
Alternatively, R⁶ and R⁷ which may be the same or different, are selected from H, C₁₋₆ alkyl, cycloalkyl, cycloalkyl C₁₋₆ alkyl, aryl, arylC₁₋₆ alkyl, heteroaryl, heteroarylC₁₋₆ alkyl, heterocyclyl, heterocydylC₁₋₆ alkyl, C₁₋₄ alkoxyC₁₋₆ alkyl, hydroxyC₁₋₆ alkyl, -(CH₂)ₙ-NR⁸R⁹, or R⁶ and R⁷ together with the atom to which they are attached form a 3, 4, 5, 6 or 7 membered saturated or unsaturated ring which may contain one or more heteroatoms selected from N, S or O, and wherein the ring may be further substituted by one or more substituents selected from halo (such as fluoro, chloro, bromo), -CN, -CF₃, -OH, -OCF₃, C₁₋₄ alkyl, C₁₋₄ alkoxy and NR⁸R⁹;
R⁸ and R⁹ which may be the same or different are selected from H or C₁₋₆ alkyl, wherein the C₁₋₆ alkyl may be further substituted by one or more substituents selected from halo (such as fluoro, chloro, bromo), -CN, -CF₃, and -OH;
and salts and solvates thereof (hereinafter "compounds of the invention").

As used herein the term "alkyl" as a group or part of a group refers to a straight or branched chain saturated aliphatic hydrocarbon radical containing the specified number(s) of carbon atoms. Such alkyl groups in particular include methyl, ethyl, n-propyl, *iso*-propyl, n-butyl, *sec*-butyl, *tert*-butyl, pentyl and hexyl.

The term "alkenyl" as a group or part of a group refers to a straight or branched chain mono- or poly-unsaturated aliphatic hydrocarbon radical containing the specified number(s) of carbon atoms. References to "alkenyl" groups include groups which may be in the E- or Z-form or mixtures thereof.

The term "alkynyl" refers to hydrocarbon groups of either straight or branched configuration with one or more carbon-carbon triple bonds which may occur at any stable point in the chain. Such alkynyl groups in particular include ethynyl, propynyl, butynyl and pentynyl.

The term "alkoxy" as a group or part of a group refers to an alkyl ether radical, wherein the term "alkyl" is defined above. Such alkoxy groups in particular include methoxy, ethoxy, n-propoxy, *iso*-propoxy, n-butoxy, *iso*-butoxy, *sec*-butoxy and *tert*-butoxy.

The term "aryl" as a group or part of a group refers to a carbocyclic aromatic radical containing the specified number(s) of carbon atoms, preferably from 5 to 14 carbon atoms, and more preferably from 5 to 10 carbon atoms, optionally substituted with one or more substituents, which may be the same or different, selected from halo (such as fluoro, chloro, bromo), -CN, -CF₃, -OH, -OCF₃, C₁₋₄ alkyl and C₁₋₄ alkoxy. Such aryl groups include cyclopentadienyl or phenyl.

The term "aryloxy" as a group or part of a group refers to an aryl ether radical, wherein the term "aryl" is defined above.

The term "cycloalkyl" as a group or part of a group refers to a saturated carbocyclic radical containing the specified number of carbon atom(s), preferably from 3 to 14 carbon atoms, more preferably 3 to 10 carbon atoms, optionally substituted with one or more substituents, which may be the same or different, selected from halo (such as fluoro, chloro, bromo), -CN, -CF₃, -OH, -OCF₃, C₁₋₄ alkyl and C₁₋₄ alkoxy. Such groups in particular include cyclobutyl and cyclohexyl.

The terms "heterocyclyl" as a group or a part of a group refers to a stable saturated or partially saturated (i.e. non-aromatic) 3 to 6 membered monocyclic ring containing one or more hetero atoms independently selected from nitrogen, oxygen and sulfur, optionally substituted with one or more substituents, which may be the same or different, selected from halo (such as fluoro, chloro, bromo), -CN, -CF₃, -OH, -OCF₃, C₁₋₄ alkyl and C₁₋₄ alkoxy.

The term "heteroaryl" as a group or part of a group refers to a stable heterocyclic aromatic 6 to 14 membered monocyclic ring containing one or more hetero atoms independently selected from nitrogen, oxygen and sulfur, optionally substituted with one or more substituents, which may be the same or different, selected from halo (such as fluoro, chloro, bromo), -CN, -CF₃, -OH, -OCF₃, C₁₋₄ alkyl and C₁₋₄ alkoxy.

The term "heteroaryloxy" as a group or part of a group refers to a heteroaryl ether radical, wherein the term "heteroaryl" is defined above.

The present invention also covers the physiologically acceptable salts of the compounds of formula (I). Suitable physiologically acceptable salts of the compounds of formula (I) include acid salts, for example sodium, potassium, calcium, magnesium and tetraalkylammonium and the like, or mono- or di- basic salts with the appropriate acid for example organic carboxylic acids such as acetic, lactic, tartaric, malic, isethionic, lactobionic and succinic acids; organic sulfonic acids such as methanesulfonic, ethanesulfonic, benzenesulfonic and p-toluenesulfonic acids and inorganic acids such as hydrochloric, sulfuric, phosphoric and sulfamic acids and the like.

The present invention also relates to solvates of the compounds of Formula (I), for example hydrates.

Compounds of formula (I) where R¹ is H may exist in tautomeric forms. The individual tautomers and mixtures thereof are included within the scope of the present invention.

Preferably, R¹ is H or C₁₋₄ alkyl, more preferably R¹ is H.

Preferably, R² is located at the C(2) position of the pyridinyl ring and represents phenyl which may be further substituted by one or more substituents, which may be the same or different, selected from halo (such as fluoro, chloro, bromo), -CN, -CF₃, -OCF₃, C₁₋₄ alkyl, -OR⁶, -O-(CH₂)ₙ-XR⁶R⁷, -O-(CH₂)ₙ-OR⁶, -O-(CH₂)ₙ-COR⁶, -O-(CH₂)ₙ-C₂₋₆ alkenyl, -O-(CH₂)ₙ-C₂₋₆ alkynyl, -(CH₂)ₙ-NR⁶R⁷, -CONR⁶R⁷, -NHCOR⁶ and -NR⁶R⁷ where n is an integer value from 1 to 6 and where X is an atom selected from C, N or S, and
where R⁶ and R⁷ which may be the same or different, are selected from H, C₁₋₆ alkyl, cycloalkyl, aryl, arylC₁₋₆ alkyl, heteroaryl, heteroarylC₁₋₆ alkyl, heterocyclyl, heterocyclylC₁₋₆ alkyl, hydroxyC₁₋₆ alkyl, -(CH₂)ₙ-NR⁸R⁹, or R⁶ and R⁷ together with the atom to which they are attached form a 3, 4, 5, 6 or 7 membered saturated or unsaturated ring which may contain one or more heteroatoms selected from N, S or O, and wherein the ring may be further substituted by one or more substituents selected from halo (such as fluoro, chloro, bromo), -CN, -CF₃, -OH, -OCF₃, C₁₋₄ alkyl and C₁₋₄ alkoxy,
or alternatively, R⁶ and R⁷ which may be the same or different, are selected from H, C₁₋₆ alkyl, cycloalkyl, cycloalkyl C₁₋₆ alkyl, aryl, arylC₁₋₆ alkyl, heteroaryl, heteroarylC₁₋₆ alkyl, heterocyclyl, heterocyclylC₁₋₆ alkyl, C₁₋₄ alkoxyC₁₋₆ alkyl, hydroxyC₁₋₆ alkyl, -(CH₂)ₙ-NR⁸R⁹, or R⁶ and R⁷ together with the atom to which they are attached form a 3, 4, 5, 6 or 7 membered saturated or unsaturated ring which may contain one or more heteroatoms selected from N, S or O, and wherein the ring may be further substituted by one or more substituents selected from halo (such as fluoro, chloro, bromo), -CN, -CF₃, -OH, -OCF₃, C₁₋₄ alkyl, C₁₋₄ alkoxy and NR⁸R⁹;
where R⁸ and R⁹ which may be the same or different are selected from H or C₁₋₆ alkyl, wherein the C₁₋₆ alkyl may be further substituted by one or more substituents selected from halo (such as fluoro, chloro, bromo), -CN, -CF₃, and -OH,
or R² is thiophenyl which may be further substituted by one or more substituents, which may be the same or different, selected from halo (such as fluoro, chloro, bromo), -CN, -CF₃, -OCF₃, C₁₋₄ alkyl and C₁₋₄ alkoxy;

More preferably, R² is located at the C(2) position of the pyridinyl ring and is phenyl which may be further substituted at the *para*-position by one or more substituents, which may the same or different, selected from halo (such as fluoro, chloro, bromo), -CN, -CF₃, -OCF₃, C₁₋₄ alkyl, -OR⁶, -O-(CH₂)ₙ-XR⁶R⁷, -O-(CH₂)ₙ-OR⁶, -O-(CH₂)ₙ-COR⁶, -O-(CH₂)ₙ-C₂₋₆ alkenyl, -O-(CH₂)ₙ-C₂₋₆ alkynyl, -(CH₂)ₙ-NR⁶R⁷, -CONR⁶R⁷, -NHCOR⁶ and -NR⁶R⁷ where n is an integer value from 1 to 6 and where X is an atom selected from C, N or S,
where R⁶ and R⁷ which may be the same or different, are selected from H, C₁₋₆ alkyl, cycloalkyl, aryl, arylC₁₋₆ alkyl, heteroaryl, heteroarylC₁₋₆ alkyl, heterocyclyl, heterocyclylC₁₋₆ alkyl, hydroxyC₁₋₆ alkyl, -(CH₂)ₙ-NR⁸R⁹, or R⁶ and R⁷ together with the atom to which they are attached form a 3, 4, 5, 6 or 7 membered saturated or unsaturated ring which may contain one or more heteroatoms selected from N, S or O, and wherein the ring may be further substituted by one or more substituents selected from halo (such as fluoro, chloro, bromo), -CN, -CF₃, -OH, -OCF₃, C₁₋₄ alkyl and C₁₋₄ alkoxy,
or alternatively, R⁶ and R⁷ which may be the same or different, are selected from H, C₁₋₆ alkyl, cycloalkyl, cycloalkyl C₁₋₆ alkyl, aryl, arylC₁₋₆ alkyl, heteroaryl, heteroarylC₁₋₆ alkyl, heterocyclyl, heterocyclylC₁₋₆ alkyl, C₁₋₄ alkoxyC₁₋₆ alkyl, hydroxyC₁₋₆ alkyl, -(CH₂)ₙ-NR⁸R⁹, or R⁶ and R⁷ together with the atom to which they are attached form a 3, 4, 5, 6 or 7 membered saturated or unsaturated ring which may contain one or more heteroatoms selected from N, S or O, and wherein the ring may be further substituted by one or more substituents selected from halo (such as fluoro, chloro, bromo), -CN, -CF₃, -OH, -OCF₃, C₁₋₄ alkyl, C₁₋₄ alkoxy and NR⁸R⁹;
and where R⁸ and R⁹ which may be the same or different are selected from H or C₁₋₆ alkyl, wherein the C₁₋₆ alkyl may be further substituted one or more substituents selected from halo (such as fluoro, chloro, bromo), -CN, -CF₃, and -OH.

Preferably, R³ is located at the C(3) or C(6) position of the pyridine ring and is selected from H, halo (such as fluoro, chloro, bromo), -CN, -CF₃, C₁₋₄ alkyl or C₁₋₄ alkoxy. More preferably R³ is H.

It will be appreciated that the present invention is intended to induce compounds having any combination of the preferred groups as listed hereinbefore.

Compounds of formula (I) which are of special interest as agents useful in the treatment or prophylaxis of disorders characterised by the overexpression of TGF-β are,
2-Phenyl-4-(3-pyridin-2-yl-1H-pyrazol-4-yl)pyridine;
2-[4-(Trifluoromethyl)phenyl]4-(3-pyridin-2-yl-1H-pyrazol-4-yl)pyridine;
2-(4-Methoxyphenyl)-4-(3-pyridin-2-yl-1H-pyrazol-4-yl)pyridine;
2-(4-Fluorophenyl)-4-(3-pyridin-2-yl-1H-pyrazol-4-yl)pyridine;
2-(4-Chlorophenyl)-4-(3-pyridin-2-yl-1H-pyrazol-4-yl)pyridine;
2-(Furan-2-yl)-4-(3-pyridin-2-yl-1H-pyrazol-4-yl)pyridine;
2-(4-Trifluoromethoxyphenyl)-4-(3-pyridin-2-yl-1H-pyrazol-4-yl)pyridine;
2-(4-Methylphenyl)-4-(3-pyridin-2-yl-1H-pyrazol-4-yl)pyridine;
2-(4-Ethylphenyl)-4-(3-pyridin-2-yl-1H-pyrazol-4-yl)pyridine;
2-(Thiophen-3-yl)-4-(3-pyridin-2-yl-1H-pyrazol-4-yl)pyridine;
2-[4-(1-Methyl-1H-imidazol-2-ylmethoxy)-phenyl]-4-(3-pyridin-2-yl-1H-pyrazol-4-yl)pyridine;
2-[4-(3-Methyl-3H-imidazol-4-ylmethoxy)-phenyl]-4-(3-pyridin-2-yl-1H-pyrazol-4-yl)-pyridine;
2-[4-(2-{1H-imidazol-1-yl}-ethoxy)-phenyl]-4-(3-pyridin-2-yl-1H-pyrazol-4-yl)pyridine;
2-[4-(2-Pyrrolidin-1-ylethoxy)-phenyl]-4-(3-pyridin-2-yl-1H-pyrazol-4-yl)pyridine;
3-({4-[4-(3-Pyridin-2-yl-1*H*-pyrazol-4-yl)pyridin-2-yl]benzyl}amino)propanenitrile;
4-{4-[4-(3-Pyridin-2-yl-1H-pyrazol-4-yl)-pyridin-2-yl]-phenyl}-morpholine;
2-[4-(1-Methyl-1-H-imidazol-4-ylmethoxy)-phenyl]-4-(3-pyridin-2-yl-1H-pyrazol-4-yl)-pyridine;
({4-[4-(3-Pyridin-2-yl-1H-pyrazol-4-yl)pyridin-2-yl]benzyl}-tetrahydro-pyran-4-yl)-amine;
{4-[4-(3-Pyridin-2-yl-1H-pyrazol-4-yl)pyridin-2-yl]benzyl}-morpholine hydrochloride;
Pyridin-3-ylmethyl-{4-[4-(3-pyridin-2-yl-1H-pyrazol-4-yl)pyridin-2-yl]benzyl}-amine;
2-Piperidin-1-yl-N-{4-[4-(3-pyridin-2-yl-1H-pyrazol-4-yl)-pyridin-2-yl]-phenyl}-acetamide;
2-Pyrrolidin-1-yl-N-{4-[4-(3-pyridin-2-yl-1H-pyrazol-4-yl)-pyridin-2-yl]-phenyl}-acetamide;
2-Morpholin-1-yl-N-{4-[4-(3-pyridin-2-yl-1H-pyrazol-4-yl)-pyridin-2-yl]-phenyl}-acetamide;
2-(4-Methyl-piperazin-1-yl)-N-{4-[4-(3-pyridin-2-yl-1H-pyrazol-4-yl)-pyridin-2-yl]-phenyl}-acetamide;
3-Piperidin-1-yl-N-{4-[4-(3-pyridin-2-yl-1H-pyrazol-4-yl)-pyridin-2-yl]-phenyl}-propionamide hydrochloride;
3-Morpholin-4-yl-N-{4-[4-(3-pyridin-2-yl-1H-pyrazol-4-yl)-pyridin-2-yl]-phenyl}-propionamide;
3-(4-Methyl-piperazin-1-yl)-N-{4-[4-(3-pyridin-2-yl-1H-pyrazol-4-yl)-pyridin-2-yl]-phenyl}-propionamide;
4-[4-(3-Pyridin-2-yl-1H-pyrazol-4-yl)-pyridin-2-yl]-N-(tetrahydro-pyran-4-yl)-benzamide;
N-(1-Ethyl-pyrrolidin-2-ylmethyl)4-[4-(3-pyridin-2-yl-1H-pyrazol-4-yl)-pyridin-2-yl]-benzamide;
(1-Ethyl)-{4-[4-(3-pyridin-2-yl-1*H*-pyrazol-4-yl)pyridin-2-yl]-phenyl}-piperazine;
(*N*-Methyl)-({4-[4-(3-pyridin-2-yl-1*H*-pyrazol-4-yl)pyridin-2-yl]-phenyl}-tetrahydro-pyran-4-yl)-amine;
({4-[4-(3-Pyridin-2-yl-1*H*-pyrazol-4-yl)pyridin-2-yl]-phenyl}-tetrahydro-pyran-4-yl)-amine;
*N*-Methyl-({4-[4-(3-pyridin-2-yl-1*H*-pyrazol-4-yl)pyridin-2-yl]benzyl}-tetrahydro-pyran-4-yl)-amine;
4-Methoxy-1-{4-[4-(3-pyridin-2-yl-1*H*-pyrazol-4-yl)pyridin-2-yl]benzyl}-piperidine;
{1-[4-(3-Pyridin-2-yl-1*H*-pyrazol-4-yl)pyridin-2-yl]benzyl}-pyrrolidine;
(1-Methyl)-{4-[4-(3-pyridin-2-yl-1*H*-pyrazol-4-yl)pyridin-2-yl]benzyl}-piperazine;
(1-Methyl-piperidin-4-yl)-{4-[4-(3-pyridin-2-yl-1*H*-pyrazol-4-yl)pyridin-2-yl]benzyl}-amine;
1-Methyl-4-{4-[4-(3-pyridin-2-yl-1*H*-pyrazol-4-yl)-pyridin-2-yl]-benzoyl}-piperazine;
4-{4-[4-(3-Pyridin-2-yl-1*H*-pyrazol-4-yl)-pyridin-2-yl]-benzoyl}-morpholine;
(4-Dimethylamino)-1-{4-[4-(3-pyridin-2-yl-1*H*-pyrazol-4-yl)-pyridin-2-yl]-benzoyl}-piperidine;
(1-Methyl)-{4-[4-(3-pyridin-2-yl-1*H*-pyrazol-4-yl)pyridin-2-yl]-phenyl}-piperazine;
{4-[4-(3-Pyridin-2-yl-1H-pyrazol-4-yl)pyridin-2-yl]benzyl}-thiomorpholine;
Dimethyl-{4-[4-(3-pyridin-2-yl-1*H*-pyrazol-4-yl)pyridin-2-yl]-benzyl}-amine;
4-[4-(3-Pyridin-2-yl-1H-pyrazol-4-yl)-pyridin-2-yl]-N-(tetrahydro-pyran-4-yl-methyl)-benzamide;
N-(2-Methoxy-ethyl)-*N*-methyl-4-[4-(3-pyridin-2-yl-1H-pyrazol-4-yl)-pyridin-2-yl]-benzamide;
N-(2-Methoxy-ethyl)-4-[4-(3-pyridin-2-yl-1H-pyrazol-4-yl)-pyridin-2-yl]-benzamide; and
N-Cyclohexylmethyl-4-[4-(3-pyridin-2-yl-1H-pyrazol-4-yl)-pyridin-2-yl]-benzamide;
and salts and solvates thereof.

Compounds of formula (I) and salts and solvates thereof may be prepared by the methodology described hereinafter, constituting a further aspect of this invention.

Compounds of formula (I) may conveniently be prepared according to the general methodologies in Schemes 1 to 11 below:

### Scheme 1

General synthetic pathway for compounds of formula (I), where R¹ is H; R² is phenyl, furanyl or thiophenyl which may be optionally substituted by one or more substituents selected from halo (such as fluoro, chloro, bromo), -CN, -CF₃, -OCF₃, C₁₋₄ alkyl, and -OR⁶ (where R⁶ is C₁₋₆ alkyl); and R³ is as hereinbefore defined: 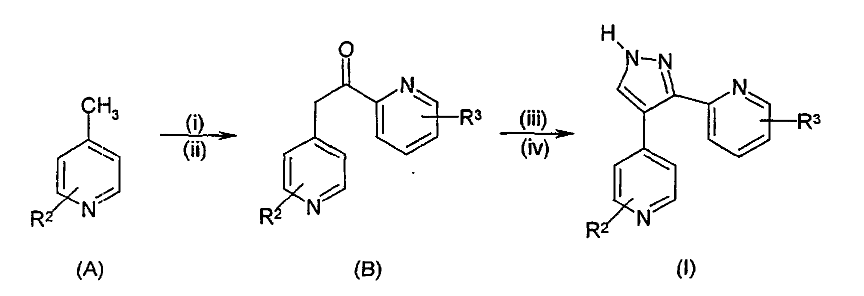 Reagents and conditions (preferred): (i) KHMDS, THF, -50°C; (ii) R³(C₅H₃N)CO₂Et, THF, -50°C; (iii) DMF.DMA, DMF, r.t.; (iv) NH₂NH₂.H₂O, DMF, r.t.

### Scheme 2

General synthetic pathway for compounds of formula (I), where R¹ is as hereinbefore defined; R² is phenyl, furanyl or thiophenyl which may be optionally substituted by one or more substituents selected from halo (such as fluoro, chloro, bromo), -CN, -CF₃, -OCF₃, C₁₋₄ alkyl, and -OR⁶ (where R⁶ is C₁₋₆ alkyl); and R³ is as hereinbefore defined:

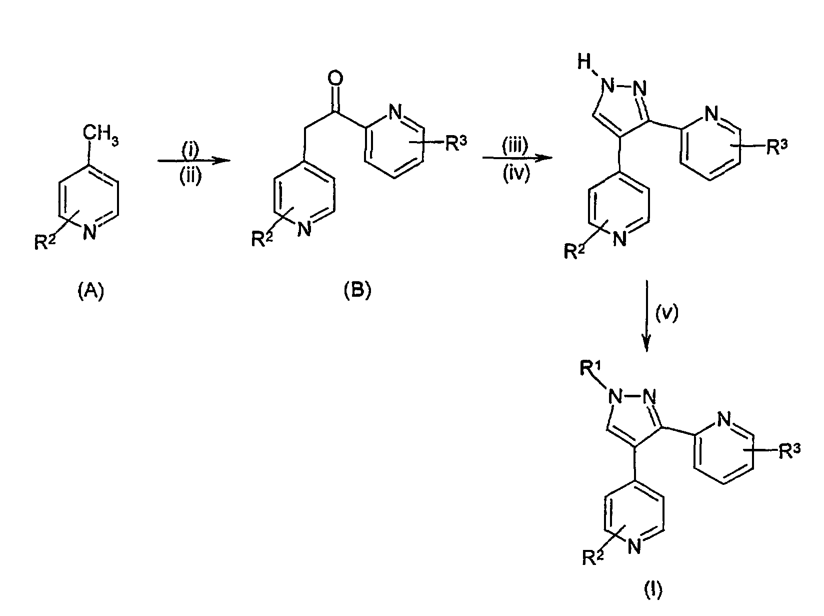

Reagents and conditions (preferred): (i) KHMDS, THF, -50°C; (ii) R³(C₅H₃N)CO₂Et, THF, -50°C; (iii) DMF.DMA, DMF, r.t.; (iv) NH₂NH₂.H₂O, DMF, r.t.; (v) R¹X, K₂CO₃, DMF, r.t.

### Scheme 3

General synthetic pathway for compounds of formula (I), where R¹ is H; R² is phenyl which may be optionally substituted by one or more substituents selected from -OR⁶, -O-(CH₂)ₙ-XR⁶R⁷, -O-(CH₂)ₙ-OR⁶, -O-(CH₂)ₙ-COR⁶, -O-(CH₂)ₙ-C₂₋₆, alkenyl, -O-(CH₂)ₙ-C₂₋₆ alkynyl (where R⁶ and R⁷ are hereinbefore defined); and R³ is as hereinbefore defined:

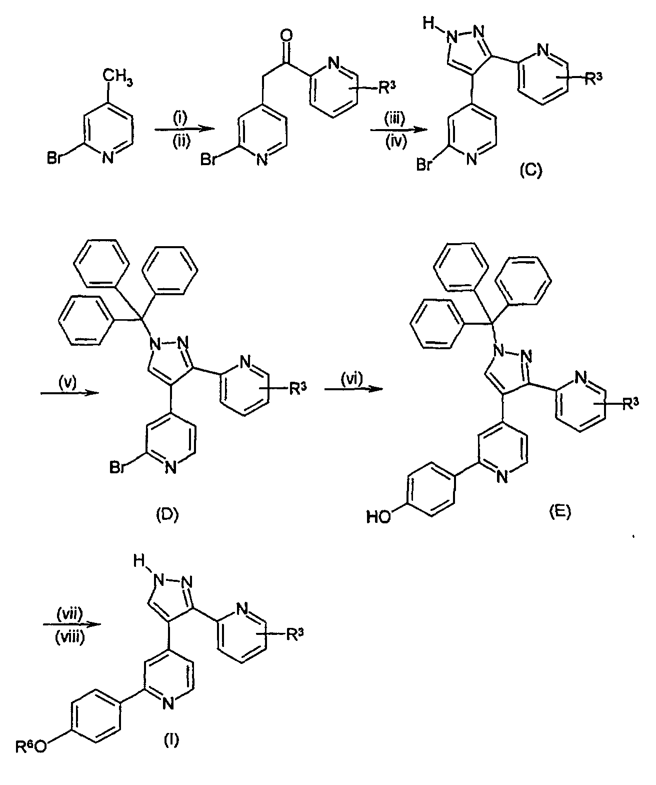

Reagents and conditions (preferred): (i) KHMDS, THF, -50°C; (ii) R³(C₅H₃N)CO₂Et, THF, -50°C; (iii) AcOH , DMF.DMA, , DMF, r.t.; (iv) NH₂NH₂.H₂O, DMF, r.t.; (v) trityl chloride, K₂CO₃, acetone, reflux; (vi) 4-Hydroxyphenylboronic acid, pinacol ester, Pd(PPh₃)₄, Na₂CO₃ (2M), toluene/EtOH, 80°C; (vii) R⁶X, K₂CO₃, acetone, reflux; (viii) HCl (N), MeOH, reflux.

It will be appreciated that as an alternative to the o-alkylation step (vii) above, a Mitsunobu-type reaction may be undertaken, the preferred reagents and conditions for which are as follows: R⁶OH, TMAD, Bu₃P, THF/CH₂Cl₂, r.t.

An alternative general synthetic pathway for the synthesis of compounds of formula (I) as described in Scheme 3, is provided below in Scheme 4.

### Scheme 4

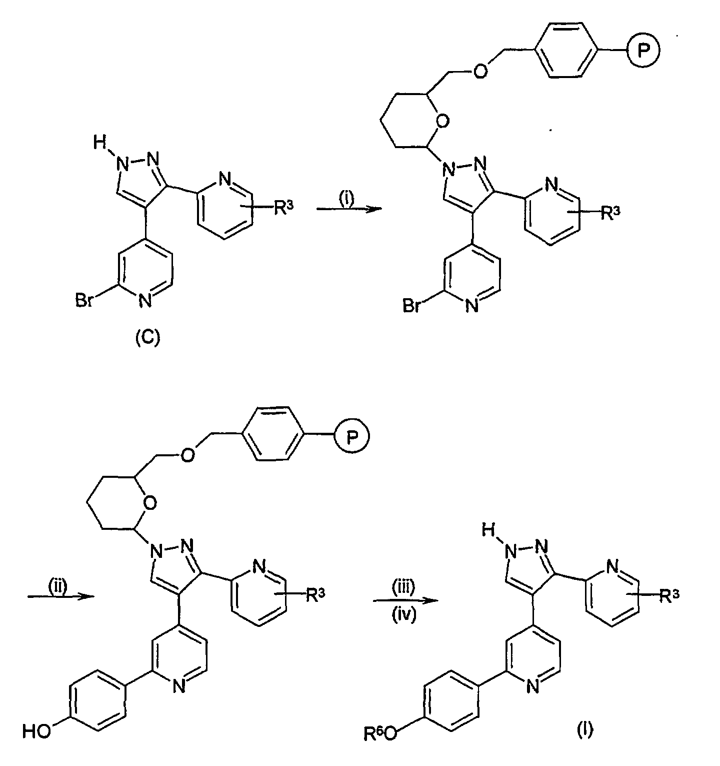

Reagents and conditions (preferred): (i) polystyrene DHP resin, PPTS, DCE, 75°C; (ii) 4-hydroxyphenylboronic acid, pinacol ester, Pd(PPh₃)₄, Na₂CO₃ (2M), toluene/EtOH, 90°C ; (iii) TMAD, Bu₃P, R⁶OH, THF/CH₂Cl₂ (1:1), r.t; (iv) 10 % TFA/CH₂Cl₂, r.t.

### Scheme 5

General synthetic pathway for compounds of formula (I), where R¹ is H; R² is phenyl substituted by -(CH₂)ₙ-NR⁶R⁷ (where R⁶ is H and R⁷ is as hereinbefore defined); and R³ is as hereinbefore defined:

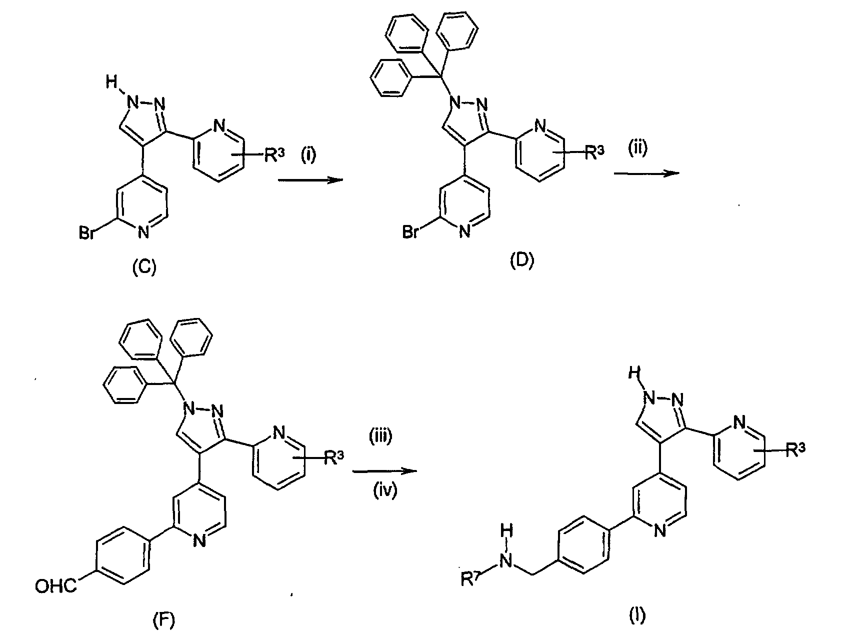

Reagents and conditions (preferred): (i) trityl chloride, K₂CO₃, acetone, reflux; (ii) 4-formylphenylboronic acid, Pd(PPh₃)₄, Na₂CO₃ (2M), toluene, reflux; (iii) R⁷NH₂, AcOH, sodium triacetoxyborohydride, DCM, r.t; (iv) TFA, CH₂Cl₂, r.t.

It will be appreciated that compounds of formula (I), where R¹ is H; R² is phenyl substituted by -(CH₂)ₙ-NR⁶R⁷ (where R⁶ and R⁷ are hereinbefore defined); and R³ is as hereinbefore defined, may be prepared by undertaking steps (i) and (ii) of Scheme 5 followed by the following additional preferred steps, (iii) R⁶R⁷N, K₂CO₃, acetone, reflux. (vi) HCl 1N, MeOH, reflux.

### Scheme 6

General synthetic pathway for compounds of formula (I), where R¹ is H; R² is phenyl substituted by -NHCOR⁶ (where R⁶ is as hereinbefore defined); and R³ is as hereinbefore defined:

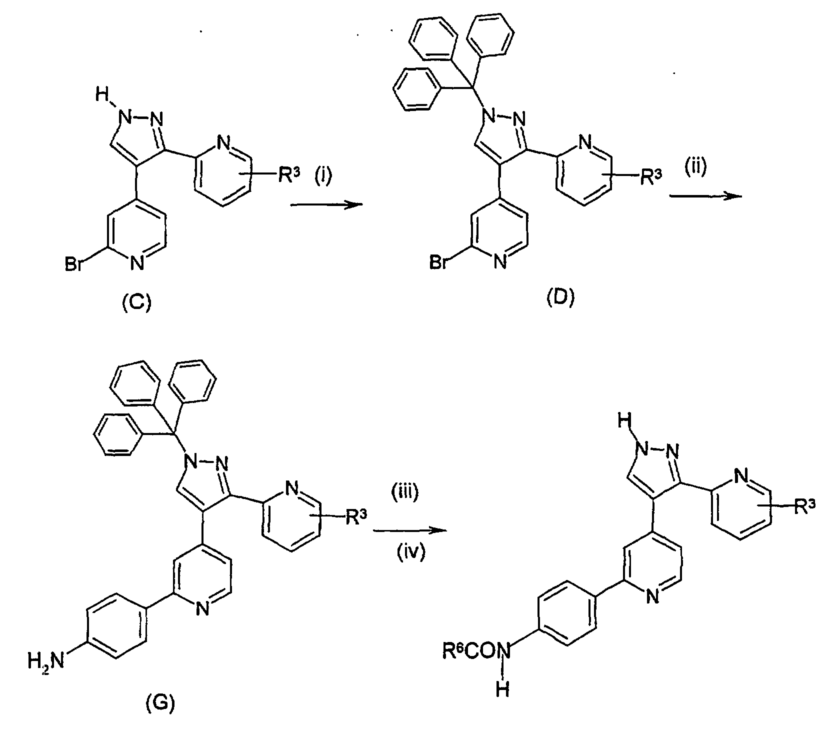

Reagents and conditions (preferred): (i) trityl chloride, K₂CO₃, acetone, reflux; (ii) 4-aminophenylboronic acid, pinacol ester, Pd(PPh₃)₄, Na₂CO₃ (2M), toluene/EtOH, 90°C;(iii) R⁶COOH, HOBT, DIEA, HBTU, DMF, r.t ;(iv) MeOH, HCl (N), reflux.

An alternative general synthetic pathway for the synthesis of compounds of formula (I) as described in Scheme 6, is provided below in Scheme 7.

### Scheme 7

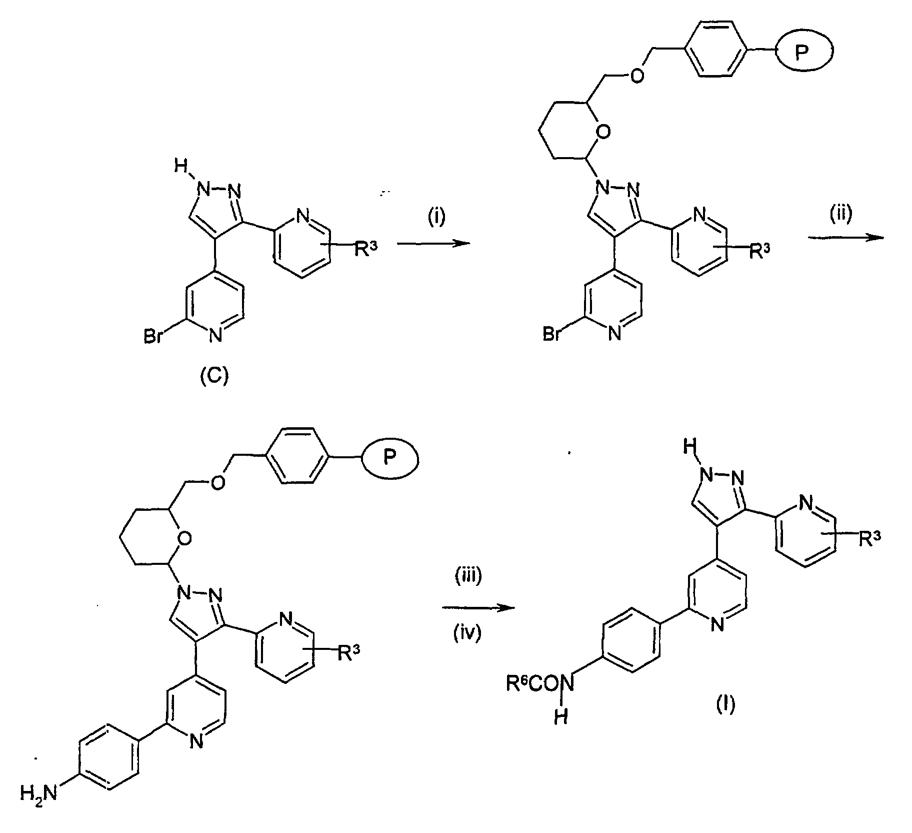

Reagents and conditions (preferred): (i) polystyrene DHP resin, PPTS, DCE, 75°C; (ii) 4-aminophenylboronic acid, pinacol ester, Pd(PPh₃)₄, Na₂CO₃ (2M), toluene/EtOH, 80°C; (iii) R⁶COOH, HOBT, DIEA, HBTU, DMF, r.t; (iv) 10 % TFA/DCM, r.t.

### Scheme 8

General synthetic pathway for compounds of formula (I), where R¹ is H; R² is phenyl substituted by -CONR⁶R⁷ (where R⁶ is H and R⁷ is as hereinbefore defined); and R³ is as hereinbefore defined:

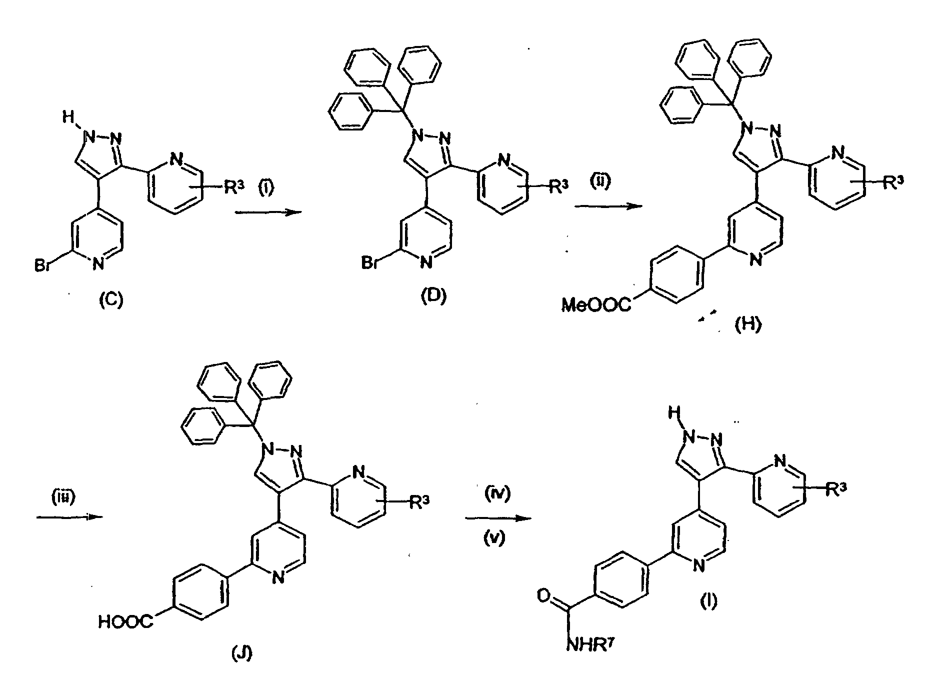

Reagents and conditions (preferred): (i) trityl chloride, K₂CO₃, acetone, reflux; (ii) 4-carboxyphenylboronic acid, methyl ester, Pd(PPh₃)₄, Na₂CO₃ (2M), toluene/EtOH, 90°C; (iii) 1N NaOH, EtOH, reflux; (iv) R⁷NH₂, HOBT, EDCl, CH₂Cl₂, r.t; (v)MeOH, HCl (N), reflux.

An alternative general synthetic pathway for the synthesis of compounds of formula (I) as described in Scheme 8, is provided below in Scheme 9.

### Scheme 9

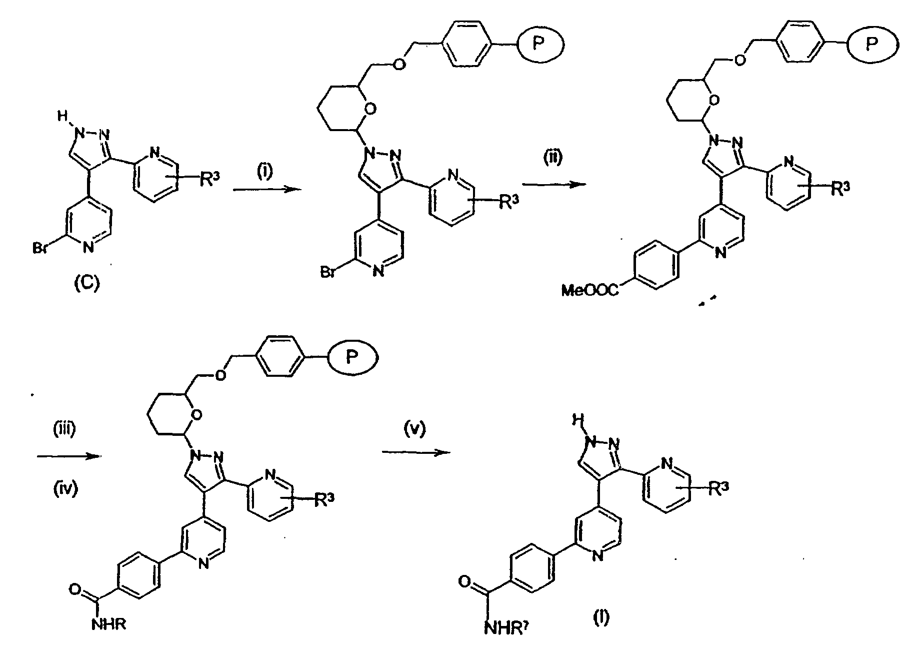

Reagents and conditions (preferred): (i) polystyrene DHP resin, PPTS, DCE, 75°C; (ii) 4-carboxyphenylboronic acid, methyl ester, Pd(PPh₃)₄, Na₂CO₃ (2M), toluene/EtOH, 90°C; (iii) 1N LiOH, DME, r.t; (iv) ) R⁷NH₂, HOBT, DIEA, HBTU, DMF, r.t; (v) MeOH, HCl N, reflux.

### Scheme 10

General synthetic pathway for compounds of formula (I), where R¹ is H; R² is phenyl substituted by NR⁶R⁷ (where R⁶ and R⁷ are as hereinbefore defined); and R³ is as hereinbefore defined:

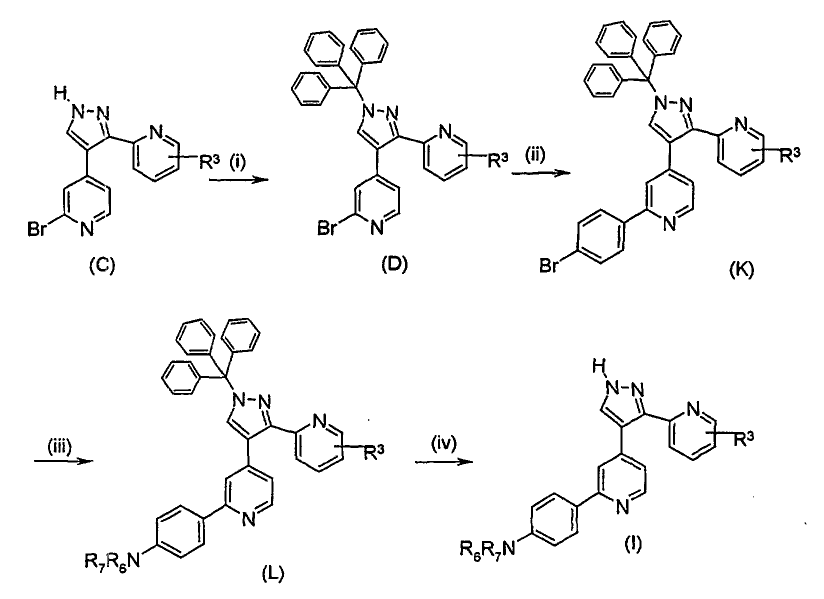

Reagents and conditions (preferred): (i) trityl chloride, K₂CO₃, acetone, reflux; (ii) 4-Bromophenylboronic acid, Pd(PPh₃)₄, Na₂CO₃ (2M), toluene/EtOH, 90°C;(iii) R⁶R⁷NH₂, Pd₂(dba)₃, binap, NaOBu^{t}, toluene, 80°C; (iv) MeOH, HCl (N), reflux.

It will be appreciated by the skilled person that in step (iii) other catalysts may be used in place of Pd₂(dba)₃. Examples of such catalysts include, but are not limited to, 1,1'-bis(diphenylphosphino)ferrocene, 1,1'-bis(diterbutyl-phosphino)ferrocene or 2'-dimethylamino-2-(dicyclohexylphosphino)1,1'-biphenyl in association with a palladium complex, or (1,5-cyc(ooctadiene)₂nickel(II) with 1,1'-bis(diphenylphosphino)ferrocene or 1,10-phenanthroline or [1,1'-bis(diphenylphosphino)ferrocene]NiCl₂ with methyl magnesium bromide.

### Scheme 11

General synthetic pathway for compounds of formula (I), where R¹ is H; R² is phenyl substituted with NR⁶R⁷ wherein R⁶ and R⁷ together with the atom to which they are attached represents morpholino; and R³ is as hereinbefore defined:

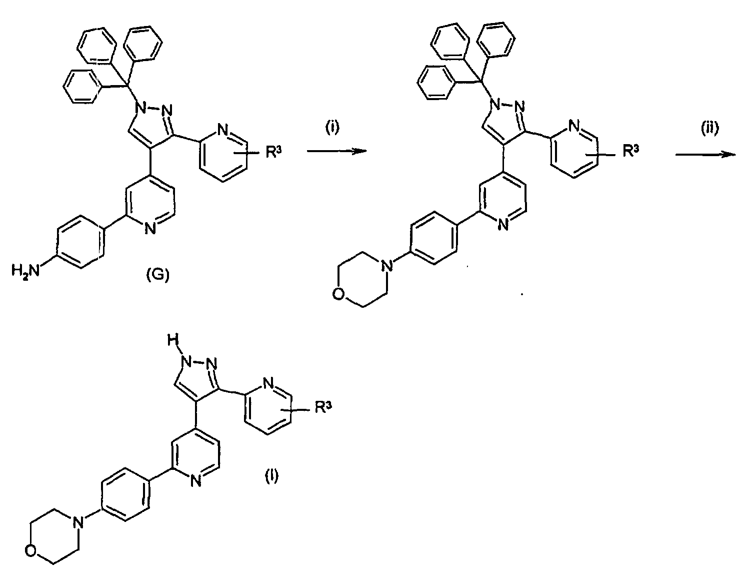

Reagents and conditions (preferred): (i) (CH₃C₆H₄SO₃CH₂CH₂)₂O, Na₂CO₃ , p-cymene, 150°C (ii) MeOH/HCl 1N (3:4), reflux.

From the same synthetic pathway it is possible to obtain the corresponding piperazine derivatives by replacing di(ethyleneglycol)di-p-tosylate with N-methylbis-(2-chloroethyl)-amine hydrochloride.

### List of Abbreviations

- DHP: Dihydropyran
- DCM: Dichloromethane
- DME: 1,2-Dimethoxyethane (YES)
- DMF.DMA: Dimethylformamide dimethylacetal
- DMF: Dimethylformamide
- DCE: Dichloroethane
- EDCI: 1-(3-Dimethylaminopropyl)-3-ethylcarbodiimide hydrochloride
- HOBT: 1-Hydroxybenzotriazole hydrate
- DIEA: N,N-Diisopropylethylamine
- HBTU: O-Benzotriazol-1-yl-N, N, N', N'-tetramethyluronium hexafluorophosphate
- KHMDS: Potassium *bis*(trimethylsilyl)amide
- PPTS: Pyridinium p-toluene sulfonate
- THF: Tetrahydrofuran
- TMAD: N,N,N',N'-(tetramethyazodicarboxamide)

A general process according to the invention for preparing a compound of formula (I) wherein R¹ is H, comprises:
(i) Addition of a suitable base, such as potassium bis(trimethylsilyl)amide or sodium bis(trimethylsilyl)amide to a substituted pyridine of formula (A), preferably in the temperature range 0 to -80°C, more preferably in the temperature range -30 to -60°C, most preferably at -50°C, in the presence of a suitable solvent such as THF;
(ii) Addition of a suitable monosubstituted pyridyl ester, R³(C₅H₃N)CO₂Et (wherein R³ is as hereinbefore defined) to the reaction mixture, preferably in the temperature range 0 to -80°C, more preferably in the temperature range -30 to -60°C, most preferably at -50°C, in the presence of a suitable solvent such as THF;
(iii) Addition of dimethylformamide dimethylacetal to the resulting ketone (B), preferably in the temperature range 0 to 75°C, more preferably in the temperature range 20 to 60°C, most preferably at room temperature, in the presence of a suitable solvent such as DMF; and
(iv) Addition of hydrazine monohydrate, NH₂NH₂.H₂O, preferably in the temperature range 0 to 75°C, more preferably in the temperature range 20 to 60°C, most preferably at room temperature, in the presence of a suitable solvent such as DMF;

In a further aspect of the present invention, compounds of formula (I) where R¹ is selected from C₁₋₄ alkyl or CH₂CONR⁴R⁵, wherein R⁴ and R⁵ are hereinbefore defined, may be prepared by N-alkylation of a pyrazole compound obtained from step (iv) above, according to the following general procedure (Scheme 2):
(v) Addition of a suitable N-alkylating reagent such as an alkyl halide, R¹X (wherein R¹ is as hereinbefore defined) in the presence of a suitable base such as potassium carbonate, sodium hydroxide or potassium hydroxide, preferably in the temperature range 0 to 75°C, more preferably in the temperature range 20 to 60°C, most preferably at room temperature, in the presence of a suitable solvent such as DMF or MeCN, followed by separation of any resulting isomeric mixture by flash chromatography on silica gel.

It will be appreciated that N-alkylation according to step (v) above may result in the formation of structural isomers of a compound of formula (I). Such isomers are afforded by N-alkylation of the tautomeric forms of a compound of formula (I) where R¹ is H, mentioned hereinbefore. The individual isomers and mixtures therof are included within the scope of the present invention.

Substituted pyridine compounds of formula (A) may be prepared by processes analogous to those known in the art (e.g. Osuch *et al., J. Org. Chem.,* 1957, **22**, 939). Examples of such preparative procedures are provided in the specific examples hereinafter.

Monosubstituted pyridyl esters, R³(C₅H₃N)CO₂Et (where R³ is as hereinbefore defined) as described in step (ii) above may be prepared by processes analogous to those known in the art. For example, where R³ = C(6)-OMe, Finger *et al*., *J. Org. Chem.,* 1962, **27**, 3965; where R³ = C(3)-OMe, Dejardin *et al., Bull. Chim. Soc. Fr.,* 1979, 289; where R³ = C(5)-Br, Chambers and Marfat, *Synth. Commun.,* 1997, **27(3)**, 515; and where R³ = C(4)-CN, Heinisch and Lotsch, *Heterocycles,* 1987, **26(3)**, 731.

N-alkylation reactions as described in step (v) may be performed according to processes analogous to those known in the art (e.g. R. Fusco, Pyrazoles, Chapter 4, p.71, *The Chemistry of Heterocyclic Compounds,* A. Weissberger (ed), Vol. 22, Intersciences, New York, 1967 and Elguero, Pyrazoles and their Benzo derivatives, p.222, *Comprehensive Heterocycles Chemistry,* A.R. Katrisky, C.W. Rees and K.T. Potts (eds), Vol. 5, Pergamon Press, Oxford, 1984).

The compounds of the present invention have been found to inhibit phosphorylation of the Smad-2 or Smad-3 proteins by inhibition of the TGF-β type 1 (Alk5) receptor.

Accordingly, the compounds of the invention have been tested in the assays described herein and have been found to be of potential therapeutic benefit in the treatment and prophylaxis of disorders characterised by the overexpression of TGF-β.

Thus there is provided a compound of formula (I) or a physiologically acceptable salt or solvate thereof for use as a medicament in human or veterinary medicine, particularly in the treatment or prophylaxis of disorders characterised by the overexpression of TGF-β.

It will be appreciated that references herein to treatment extend to prophylaxis as well as the treatment of established conditions. It will further be appreciated that references herein to treatment or prophylaxis of disorders characterised by the overexpression of TGF-β, shall include the treatment or prophylaxis of TGF-β associated disease such as fibrosis, especially liver and kidney fibrosis, cancer development, abnormal bone function and inflammatory disorders, and scarring.

Other pathological conditions which may be treated in accordance with the invention have been discussed in the introduction hereinbefore. The compounds of the present invention are particularly suited to the treatment of fibrosis and related conditions.

Compounds of the present invention may be administered in combination with other therapeutic agents, for example antiviral agents for liver diseases, or in combination with ACE inhibitors or Angiotensin II receptor antagonists for kidney diseases.

According to another aspect of the invention, there is provided the use of a compound of formula (I) or a physiologically acceptable salt or solvate thereof for the manufacture of a medicament for the treatment and/or prophylaxis of disorders characterised by the overexpression of TGF-β, particularly fibrosis.

Compounds of the invention may be formulated for administration in any convenient way, and the invention therefore also includes within its scope pharmaceutical compositions for use, in therapy, comprising a compound of formula (I) or a physiologically acceptable salt or solvate thereof in admixture with one or more physiologically acceptable diluents or carriers.

There is also provided according to the invention a process for preparation of such a pharmaceutical composition which comprises mixing the ingredients.

Compounds of the invention may, for example, be formulated for oral, buccal, parenteral, topical or rectal administration.

Tablets and capsules for oral administration may contain conventional excipients such as binding agents, for example syrup, acacia, gelatin, sorbitol, tragacanth, mucilage of starch or polyvinyl pyrrolidone; fillers, for example, lactose, microcrystalline cellulose, sugar, maize- starch, calcium phosphate or sorbitol; lubricants, for example. magnesium stearate, stearic acid, talc, polyethylene glycol or silica; disintegrants, for example, potato starch, croscarmellose sodium or sodium starch glycollate; or wetting agents such as sodium lauryl sulphate. The tablets may be coated according to methods well known in the art. Oral liquid preparations may be in the form of, for example, aqueous or oily suspensions, solutions, emulsions, syrups or elixirs, or may be presented as a dry product for constitution with water or other suitable vehicle before use. Such liquid preparations may contain conventional additives such as suspending agents, for example, sorbitol syrup, methyl cellulose, glucose/sugar syrup, gelatin, hydroxymethyl cellulose, carboxymethyl cellulose, aluminium stearate gel or hydrogenated edible fats; emulsifying agents, for example, lecithin, sorbitan mono-oleate or acacia; non-aqueous vehicles (which may include edible oils), for example almond oil, fractionated coconut oil, oily esters, propylene glycol or ethyl alcohol; or preservatives, for example, methyl or propyl p- hydroxybenzoates or sorbic acid. The preparations may also contain buffer salts, flavouring, colouring and/or sweetening agents (e.g. mannitol) as appropriate.

For buccal administration the compositions may take the form of tablets or lozenges formulated in conventional manner.

The compounds may also be formulated as suppositories, e.g. containing conventional suppository bases such as cocoa butter or other glycerides.

Compounds of the invention may also be formulated for parenteral administration by bolus injection or continuous infusion and may be presented in unit dose form, for instance as ampoules, vials, small volume infusions or pre-filled syringes, or in multidose containers with an added preservative. The compositions may take such forms as solutions, suspensions, or emulsions in aqueous or non-aqueous vehicles, and may contain formulatory agents such as anti-oxidants, buffers, antimicrobial agents and/or toxicity adjusting agents. Alternatively, the active ingredient may be in powder form for constitution with a suitable vehicle, e.g. sterile, pyrogen-free water, before use. The dry solid presentation may be prepared by filling a sterile powder aseptically into individual sterile containers or by filling a sterile solution aseptically into each container and freeze-drying.

By topical administration as used herein, we include administration by insufflation and inhalation. Examples of various types of preparation for topical administration include ointments, creams, lotions, powders, pessaries, sprays, aerosols, capsules or cartridges for use in an inhaler or insufflator or drops (e.g. eye or nose drops).

Ointments and creams may, for example, be formulated with an aqueous or oily base with the addition of suitable thickening and/or gelling agents and/or solvents. Such bases may thus, for example, include water and/or an oil such as liquid paraffin or a vegetable oil such as arachis oil or castor oil or a solvent such as a polyethylene glycol. Thickening agents which may be used include soft paraffin, aluminium stearate, cetostearyl alcohol, polyethylene glycols, microcrystalline wax and beeswax.

Lotions may be formulated with an aqueous or oily base and will in general also contain one or more emulsifying agents, stabilising agents, dispersing agents, suspending agents or thickening agents.

Powders for external application may be formed with the aid of any suitable powder base, for example, talc, lactose or starch. Drops may be formulated with an aqueous or non-aqueous base also comprising one or more dispersing agents, solubilising agents or suspending agents.

Spray compositions may be formulated, for example, as aqueous solutions or suspensions or as aerosols delivered from pressurised packs, with the use of a suitable propellant, e.g. dichlorodifluoromethane, trichlorofluoromethane, dichlorotetrafluoroethane, 1,1,1,2,3,3,3-heptafluoropropane, 1,1,1,2- tetrafluorethane, carbon dioxide or other suitable gas.

Capsules and cartridges for use in an inhaler or insufflator, of for example gelatin, may be formulated containing a powder mix of a compound of the invention and a suitable powder base such as lactose or starch.

Compounds of the invention may conveniently be administered in amounts of, for example, 0.01 to 100mg/kg body weight, suitably 0.05 to 25mg/kg body weight orally, one or more times a day. The precise dose will of course depend on the age and condition of the patient, the particular route of administration chosen, and is entirely within the discretion of the administering physician.

The following non-limiting Examples illustrate the present invention.

### Intermediates

### Compounds of Formula (A)

### Intermediate A1: 2-Phenyl-4-methyl-pyridine

2-Bromo-4-methylpyridine (ALDRICH, 10 g, 58 mmol) was dissolved in toluene (150ml) and lithium chloride (3 eq, 7.4 g) and tetrakis(triphenylphosphine)palladium (2 mol%, 1.343 g) added under N₂ and degassed. The mixture was cooled in an ice bath and aqueous sodium carbonate (2M, 2.5 eq) added slowly and stirred for 10min. A solution of phenylboronic acid (14.17 g, 2 eq) in ethanol (60 ml) was added slowly and stirred for 3min, returned to room temp and allowed to stir at rt for 30 min, then heated to reflux for 10 h. The mixture was allowed to cool, the phases separated and the aqueous phase extracted with further toluene (2x50 ml). The combined organic phases were washed with 50ml NaOH (1N) then extracted with 1N HCl (2x100 ml). These combined acid extracts were washed with toluene (50 ml) and then basified to pH11 with aq NaOH with cooling. The product was then extracted into CH₂Cl₂ (3x100 ml) dried and filtered to give, on concentration, the title compound as a yellow oil (75.3%).
TLC SiO₂ CH₂Cl₂ Rf 0.15
GC-MS 169

### Intermediate A2: 2-(4-Trifluoromethylphenyl)-4-methyl-pyridine

A procedure similar to that for Intermediate A1, with substitution of 4-trifluoromethylphenylboronic acid (Lancaster,10 g ) for phenylboronic acid gave the title compound as a yellow oil (3.66 g).
TLC SiO₂ CH₂Cl₂ Rf 0.5
GC-MS: 237

### Intermediate A3: 2-(4-Methoxyphenyl)-4-methyl-pyridine

A procedure similar to that for Intermediate A1, with substitution of 4-methoxyphenylboronic acid (Lancaster,15 g) for phenylboronic acid gave the title compound as an orange oil (14.2 g).
TLC SiO₂ CH₂Cl₂/MeOH 98/2 Rf 0.85
[APCI MS] m/z 200 (MH+)

### Intermediate A4: 2-(4-Fluorophenyl)-4-methyl-pyridine

A procedure similar to that for Intermediate A1, with substitution of 4-fluorophenylboronic acid (Lancaster,11 g) for phenylboronic acid gave the title compound as a yellow oil (6.5 g).
TLC SiO₂ CH₂Cl₂ Rf 0.20
GC-MS: 187

### Intermediate A5: 2-(4-Chlorophenyl)-4-methyl-pyridine

A procedure similar to that for Intermediate A1, with substitution of 4-chlorophenylboronic acid (Lancaster, 2.5 g) for phenylboronic acid gave the title compound as a yellow solid (2.6 g).
m.p 63°C
TLC SiO₂ CH₂Cl₂/EtOAc 95/5 Rf 0.64
GC-MS: 203

### Intermediate A6: 2-Furan-2-yl-4-methyl-pyridine

A procedure similar to that for Intermediate A1, with substitution of furan-2-boronic acid (Lancaster, 5 g) for phenylboronic acid gave the title compound as an oil (1.6 g).
TLC SiO₂ CH₂Cl₂/EtOAc 98/2 Rf 0.48
GC-MS: 159

### Intermediate A7: 2-(4-Trifluoromethoxyphenyl)-4-methyl-pyridine

A procedure similar to that for Intermediate A1, with substitution of 4-trifluoromethoxyphenylboronic acid (Lancaster, 5 g) for phenylboronic acid gave the title compound as an oil (3.2 g).
TLC SiO₂ CH₂Cl₂/EtOAc 95/5 Rf 0.54
GC-MS: 253

### Intermediate A8: 2-(4-Methylphenvi)-4-methyl-pyridine

A procedure similar to that for Intermediate A1, with substitution of 4-methylphenylboronic acid (Fluka, 5.97 g) for phenylboronic acid gave the title compound as an oil (5.7 g).
TLC SiO₂ CH₂Cl₂/EtOAc 95/5 Rf 0.6
GC-MS: 183

### Intermediate A9: 2-(4-Ethylphenyl)-4-methyl-pyridine

A procedure similar to that for Intermediate A1, with substitution of 4-ethylphenylboronic acid (Lancaster, 4 g) for phenylboronic acid gave the title compound as an oil (3.9 g).
TLC SiO₂ CH₂Cl₂/EtOAc 95/5 Rf 0.50
GC-MS: 197

### Compounds of formula (B)

### Intermediate B1: 2-(2-Phenylpyridin-4-yl)-1-pyridin-2-yl-ethanone

To a solution of Intermediate A1 (2-phenyl-4-methyl-pyridine, 20.34 g ) in dry THF (250 ml) at -50°C under argon, a solution of potassium bis-(trimethylsilyl)amide 0.5M in toluene (265 ml) was added dropwise. The yellow solution was stirred at this temperature for 30 min. Then a solution of ethyl picolinate (20 g) in dry THF (20 ml) was added and the reaction mixture was allowed to warm up to room temperature and stirred for 18 h. The solvent was evaporated under reduced pressure and the solid residue was triturated with diethyl ether, filtered and washed with diethyl ether. The solid was then diluted with saturated NH₄Cl solution and the aqueous phase was extracted with ethyl acetate. The organic layer was dried over sodium sulfate and concentrated. The crude product was purified by recrystallisation from ⁱPrOH (60 ml), filtration and washing with pentane to give, after drying, the title compound as a solid (16.4 g).
m.p 106°C
TLC SiO₂ CH₂Cl₂/MeOH 98/2 Rf 0.18
[APCI MS] m/z 275(MH+)

### Intermediate B2: 1-Pyridin-2-yl-2-[2-(4-trifluoromethyl-phenyl)-pyridin-4-yl]-ethanone

A procedure similar to that for Intermediate B1, with substitution of Intermediate A2 (3.6 g) for Intermediate A1, gave the title compound as a pale yellow solid (1.96 g).
m.p 95°C
TLC SiO₂ CH₂Cl₂/MeOH 98/2 Rf 0.5
[APCI MS] m/z 343(MH+)

### Intermediate B3: 2-[2-(4-Methoxy-phenyl)-pyridin-4-yl]-1-pyridin-2-yl-ethanone

A procedure similar to that for Intermediate B1, with substitution of Intermediate A3 (5.0 g) for Intermediate A1, gave the title compound as a pale yellow solid (2.0 g).
m.p 86°C
TLC SiO₂ cyclohexane/EtOAc 70/30 Rf 0.37
[APCI MS] m/z 305 (MH+)

### Intermediate B4: 2-[2-(4-Fluoro-phenyl)-pyridin-4-yl]-1-pyridin-2-yl-ethanone

A procedure similar to that for Intermediate B1, with substitution of Intermediate A4 (6.5 g) for Intermediate A1, gave the title compound as a dark yellow oil (3.1 g).
TLC SiO₂ CH₂Cl₂/MeOH 95/5 Rf 0.30
[APCI MS] m/z 293(MH+)

### Intermediate B5: 2-[2-(4-Chloro-phenyl)-pyridin-4-yl]-1-pyridin-2-yl-ethanone

A procedure similar to that for Intermediate B1, with substitution of Intermediate A5 (2.6 g) for Intermediate A1, gave the title compound as a dark red oil (2.6 g).
TLC SiO₂ CH₂Cl₂/MeOH 98/2 Rf 0.42
[APCI MS] m/z 309(MH+)

### Intermediate B6: 2-(2-Furan-2-yl-pyridin-4-yl)-1-pyridin-2-yl-ethanone

A procedure similar to that for Intermediate B1, with substitution of Intermediate A6 (1.6 g) for Intermediate A1, gave the title compound as a yellow oil (1.6 g).
TLC SiO₂ CH₂Cl₂/MeOH 98/2 Rf 0.32
GC-MS: 264

### Intermediate B7: 2-[2-(4-Trifluoromethoxy-phenyl)-pyridin-4-yl]-1-pyridin-2-yl-ethanone

A procedure similar to that for Intermediate B1, with substitution of Intermediate A7 (3.2 g) for Intermediate A1, gave the title compound as a yellow oil (2.5 g).
TLC SiO₂ CH₂Cl₂/AcOEt 95/5 Rf 0.30
GC-MS 358

### Intermediate B8: 2-[2-(4-Methylphenyl)-pyridin-4-yl]-1-pyridin-2-yl-ethanone

A procedure similar to that for Intermediate B1, with substitution of Intermediate A8 (5.7 g) for Intermediate A1, gave the title compound as a yellow solid (1.9 g).
m.p 103°C
TLC SiO₂ CH₂Cl₂/MeOH 98/2 Rf 0.2

### Intermediate B9: 2-[2-(4-Ethylphenyl)-pyridin-4-yl]-1-pyridin-2-yl-ethanone

A procedure similar to that for Intermediate B1, with substitution of Intermediate A9 (3.9 g) for Intermediate A1, gave the title compound as a yellow oil (1.2 g).
TLC SiO₂ CH₂Cl₂/EtOAc 95/5 Rf 0.32
GC-MS 302

### Intermediate B10: 2-(2-Thiophen-3-yl-pyridin-4-yl)-1-pvridin-2-yl-ethanone

A procedure similar to that for Intermediate B1, with substitution of 2-thiophen-3-yl-4-methyl-pyridine (HighForceResearch, 5 g) for Intermediate A1, gave the title compound as a yellow oil (1.1 g).
TLC SiO₂ CH₂Cl₂/MeOH 95/5 Rf 0.24
[APCI MS] m/z 281(MH+)

### Intermediate B11: 2-[2-Bromo-pyridin-4-yl]-1-pyridin-2-yl-ethanone

To a solution of 2-bromo-4-methyl-pyridine (ALDRICH, 27 g) in dry THF (270 ml) was added ethyl picolinate (28.5 g). The resulting mixture was cooled to -78°C under argon and a solution of sodium bis-(trimethylsilyl)amide 1M in THF (345 ml) was added dropwise at -78°C. The resulting reaction mixture was allowed to reach room temperature and subsequently stirred overnight. The solvent was evaporated under reduced pressure and the solid residue triturated with diethyl ether, filtered and washed with diethyl ether. The solid was then diluted with saturated NH₄Cl solution and the aqueous phase extracted with ethyl acetate. The organic layer was dried over sodium sulfate and concentrated. The resulting orange powder was washed with pentane to give the title compound as a yellow solid (33.97 g).
TLC SiO₂ CH₂Cl₂/MeOH 95/5 Rf 0.8
m.p 111.2°C

### Intermediate B12: 2-[2-Bromo-pyridin-4-yl]-1-(6-methyl-pyridin-2-yl)-ethanone

2-Bromo-4-methyl-pyridine (Aldrich, 5g, 29 mmol) and methyl-6-methylpicolinate (1.1 eq, 4.82 g, 32 mmol) were reacted as described for intermediate B11 to afford the title compound as a yellow solid (5.84 g, 70%).
TLC SiO₂ CH₂Cl₂/MeOH 98/2 Rf 0.38
[APCI MS] m/z: 292 (MH+)

### Intermediate B13: 2-[2-(4-Isopropylphenyl)-pyridin-4-yl]-1-pyridin-2-yl-ethanone

A procedure similar to that for Intermediate B1, with substitution of 2-isopropylphenyl-4-methyl-pyridine (2.2g) for Intermediate A1, gave the title compound as a brown oil (1.9 g, 57.7%).
TLC SiO₂ CH₂Cl₂/MeOH 98/2 Rf 0.46
MS m/z 316

### Compounds of Formula (C)

### Intermediate C1: 2-Bromo-4-(3-pyridin-2-yl-1H-pyrazol-4-yl)pyridine

A solution of 2-(2-bromo-pyridin-4-yl)-1-pyridin-2-yl-ethanone (69.68g, 0.251 mol) in dry DMF (13.5 ml) under nitrogen was treated with glacial acetic acid (2.4eq, 35ml, 0.61 mol) over 2 min. DMF.DMA (1.5eq., 50 ml, 0.376 mol) was subsequently added dropwise and the mixture stirred at room temperature under nitrogen for 78 min. Hydrazine monohydrate (7.5eq, 91 ml, 1.876 mol) was added dropwise, and the resulting mixture was heated at 50°C for 2 h and then allowed to stir overnight at room temperature. The reaction mixture was poured into water (4 l) and extracted with ethyl acetate (1 l). The organic extracts were dried over Na₂SO₄ and filtered. After concentration in vacuo a brown oil was afforded, which was crystallised from acetonitrile to produce a cream solid (45.8 g, 60.5%).
¹H NMR (CDCl₃): δ 8.62 (d,1H); 8.28 (d, 1H); 7.68 (s, 1H); 7.62 (td,1H); 7.51 (s, 1H); 7.39 (d, 1H); 7.26-7.22 (m,2H).

### Intermediate C2: 2-Bromo-4-[3-(6-methyl-pyridin-2-yl)-1H-pyrazol-4-yl]pyridine

2-(2-Bromo-pyridin-4-yl)-1-(6-methyl-pyridin-2-yl)-ethanone (5.84 g, 20 mmol) was reacted as described for intermediate C1 to afford the title compound as a yellow solid (3.07 g, 49%).
[APCI MS] m/z: 315 (MH+)

### Compounds of Formula (D)

### Intermediate D1: 2-Bromo-4-(3-pyridin-2-yl-1-trityl-1H-pyrazol-4-yl)pyridine

To a solution of 2-bromo-4-(3-pyridin-2-yl-1*H-*pyrazol-4-yl)pyridine (1eq, 35.2 mmol, 10.6 g) in acetone (200 ml) was added potassium carbonate (3eq, 14.6 g) and trityl chloride (1.5eq, 53.24mmol, 14.8g). The reaction mixture was subsequently heated to reflux and stirred for a further 24h. The reaction mixture was filtered and the filtrate concentrated, and then partitioned between CH₂Cl₂ and H₂O. The organic phase was dried over Na₂SO₄ and concentrated. The resulting crude material was purified by flash chromatography, eluting with CH₂Cl₂/MeOH (98:2) to give the title compound as a white solid (16.3 g, 85.3%), which contains the 2-trityl isomer as a minor component.
m.p 202°C
[APCI MS] m/z 544 (MH+)

### Intermediate D2: 2-Bromo-4-[3-(6-methyl-pyridin-2-yl)-1-trityl-1H-pyrazol-4-yl]pyridine

2-Bromo-4-[3-(6-methyl-pyridin-2-yl)-1*H*-pyrazol-4-yl]pyridine (3.07 g , 9.8 mmol) and trityl chloride (1.5 eq, 4.1 g, 14.7 mmol) were reacted as described for Intermediate D1 to afford the title compound as the major isomer of a mixture of the two regioisomers , as a light yellow solid (4.9 g, 90%).
[APCI MS] m/z 558 (MH+)

### Intermediate E: 2-(4-Hydroxy-phenyl)-4-(3-pyridin-2-yl-1-trityl-1H-pyrazol-4-yl)-pyridine

A solution of 2-bromo-4-(3-pyridin-2-yl-1-trityl-1*H-*pyrazol-4-yl)pyridine (6.51 g, 12 mmol) in toluene (150 mL) was treated with tetrakis triphenylphosphine palladium (532 mg) and stirred at room temperature for 30 min. Na₂CO₃ (2M) (31.8 ml) was added to the reaction mixture, followed by 4-hydroxyphenyl boronic acid, pinacol ester (2.03eq, 6.08 g, 27.6 mmol) in EtOH (60 ml). The resulting mixture heated under reflux overnight. The cooled mixture was poured into ice and extracted with toluene. The organic layer was washed with water, dried over Na₂SO₄ and filtered. Evaporation of the solvent *in vacuo* gave a crude oil which was purified by chromatography on silica gel (CH₂Cl₂/MeOH 97:3) to give the title compound as a white solid (5.9 g, 88.4%), which contains the 2-trityl isomer as a minor component.
¹H NMR (300 MHz, CDCl₃) δ: 8.72 (m, 1H); 8.63 (m, 1H); 7.94-7.7 (m, 5H); 7.52 (m, 8H); 7.44 (m,7H); 7.37(m, 1H); 7.24 (m, 1H); 7.24 ( m, 1H); 6.90 (m, 2H).

### Intermediate F: 4-[4-(3-Pyridin-2-yl-1-trityl-1H-pyrazol-4-yl)pyridin-2-yl]benzaldehyde

A solution of 2-bromo-4-(3-pyridin-2-yl-1-trityl-1*H*-pyrazol-4-yl)pyridine (5g, 9.2mmol) in toluene (200 ml) was treated with tetrakis triphenylphosphine palladium (532 mg) and stirred at room temperature for 20 min. Aqueous Na₂CO₃ (30 ml) was added, followed by 4-formylphenylboronic acid (2eq, 2.76 g) and the mixture heated under reflux overnight. The cooled mixture was diluted with ethyl acetate and washed with water (x3). This gave a brown foam which was purified by Biotage MPLC with ethyl acetate : cyclohexane (5:1) as eluant. This gave the title compound as a white solid (4.44 g, 85%), containing the 2-trityl isomer as a minor component.
[APCl MS] m/z 569 (MH+)

### Intermediate G: 4-[4-(3-Pyridin-2-yl-1-trityl-1H-pyrazol-4-yl)pyridin-2-yl]phenyl-amine

2-Bromo-4-(3-pyridin-2-yl-1-trityl-1*H*-pyrazol-4-yl)pyridine and 4-aminophenyl-boronic acid, pinacol ester were coupled as described for Intermediate E1 to afford the title compound as the major isomer of a mixture of the two regioisomers (8g, 72%), white solid.
[APCIMS] m/z: 556.2 (MH+)

### Intermediate H: 4-[4-(3-Pyridin-2-yl-1-trityl-1H-pyrazol-4-yl)pyridin-2-yl]-benzoic acid methyl ester

2-Bromo-4-(3-pyridin-2-yl-1-trityl-1*H*-pyrazol-4-yl)pyridine and 4-(methoxy-carbonyl-phenyl)boronic acid were coupled as described for Intermediate E to afford the title compound as the major isomer of a mixture of the two regioisomers (4g, 40%).
[APCl MS] m/z: 599 (MH+)

### Intermediate J: 4-[4-(3-Pyridin-2-yl-1-trityl-1H-pyrazol-4-yl)pyridin-2-yl]-benzoic acid

To a solution of Intermediate H (4g, 6.68mmol) in EtOH (100ml) was added NaOH 1N (2eq., 13.38ml). The reaction mixture was refluxed overnight. The solvent was removed under reduced pressure and the residue treated with NH₄Cl (sat.). After extraction with diethyl ether, the organic phase was dried over Na₂SO₄, filtered and evaporated to dryness to afford the title compound as the major isomer of the mixture of the two regioisomers (3.9g, 99.7%).
[APCI MS] m/z: 585 (MH+)

### Intermediate K1: 2-(4-Bromo-phenyl)-4-(3-pyridin-2-yl-1-trityl-1H-pyrazol-4-yl) pyridine

2-Bromo-4-(3-pyridin-2-yl-1-trityl-1*H*-pyrazol-4-yl)pyridine (6 g 11 mmol) and 4-bromo-phenyl-boronic acid were coupled as described for Intermediate E to afford the title compound as the major isomer of a mixture of the two regioisomers (5.85 g, 86%).
[APCI MS] m/z 621 (MH+)

### Examples

### Example 1: 2-Phenyl-4-(3-pyridin-2-yl-1H-pyrazol-4-yl)pyridine

To a solution of Intermediate B1 (10.00 g) in DMF (60 ml) at rt under N₂ was added dimethylformamide dimethylacetal (3 eq., 14.53ml, ALDRICH ). After stirring for 7h, hydrazine monohydrate (35 eq, 60 ml, ALDRICH) was added over 10min then the mixture warmed to 50°C for 2h and allowed to stand at room temperature overnight. Water (500 ml) was added and extracted with CH₂Cl₂ (3x200 ml). These organic phases were combined and washed with brine (200 ml) before being dried, concentrated and the residue purified by column chromatography on silica gel with CH₂Cl₂ then CH₂Cl₂/ MeOH 98:2. This gave the title compound as an off-white powder (6.28 g, 58%).
mp. 148°C
TLC SiO₂ CH₂Cl₂/ MeOH 90/10 Rf 0.38
[APCI MS] m/z 299 (MH+)

### Example 2: 2-[4-(Trifluoromethyl)phenyl]-4-(3-pyridin-2-yl-1H-pyrazol-4-yl) pyridine

A procedure similar to that for Example 1 using Intermediate B2 (1.94 g) gave, after crystallisation from ⁱPrOH/ⁱPr₂O, the title compound as white crystals (140 mg).
m.p 165°C
TLC SiO₂ CH₂Cl₂/ MeOH 90/10 Rf 0.53
[APCI MS] m/z 367(MH+)

### Example 3: 2-(4-Methoxyphenyl)-4-(3-Pyridin-2-yl-1H-pyrazol-4-yl)pyridine

A procedure similar to that for Example 1 using Intermediate B3 (1.0 g) gave, after chromatography, the title compound as a yellow semi-solid (270 mg).
TLC SiO₂ CH₂Cl₂/ MeOH 95/5 Rf 0.17
[APCI MS] m/z 329(MH+)

### Example 4: 2-(4-Fluorophenyl)-4-(3-pyridin-2-yl-1H-pyrazol-4-yl)pyridine

A procedure similar to that for Example 1 using Intermediate B4 (1.50 g) gave, after crystallisation from ⁱPrOH, the title compound as white crystals (280 mg).
m.p 117°C
TLC SiO₂ CH₂Cl₂/ MeOH 95/5 Rf 0.12.
[APCI MS] m/z 317(MH+)

### Example 5: 2-(4-Chlorophenyl)-4-(3-pyridin-2-yl-1H-pyrazol-4-yl)pyridine

A procedure similar to that for Example 1 using Intermediate B5 (2 g) gave, on precipitation from CH₂Cl₂/hexane, the title compound as a white solid (100 mg).
m.p 127-129°C
TLC SiO₂ CH₂Cl₂/MeOH 95/5 Rf 0.34
[APCI MS] m/z 333(MH+)

### Example 6:2-(Furan-2-yl)-4-(3-pyridin-2-yl-1H-pyrazol-4-yl)pyridine

A procedure similar to that for Example 1 using Intermediate B6 (2 g) gave, after recrystallisation from EtOAc, the title compound as white crystals (90 mg).
m.p 135-137°C
TLC SiO₂ CH₂Cl₂/MeOH 90/10 Rf 0.56
[APCI MS] m/z 289(MH+)

### Example 7: 2-(4-Trifluoromethoxyphenyl)-4-(3-pyridin-2-yl-1H-pyrazol-4-yl)pyridine

A procedure similar to that for Example 1 using Intermediate B7 (2.2 g) gave, after precipitation from CH₂Cl₂/hexane, the title compound as a white solid (380 mg).
m.p 138°C
TLC SiO₂ Toluene/isopropylamine 90/10 Rf 0.26
[APCI MS] m/z 383(MH+)

### Example 8: 2-(4-Methylphenyl)-4-(3-pyridin-2-yl-1H-pyrazol-4-yl)pyridine

A procedure similar to that for Example 1 using Intermediate B8 (1.4 g) gave, after precipitation from CH₂Cl₂/hexane, the title compound as a white solid (600 mg).
m:p 94°C
TLC SiO₂ Toluene/sopropylamine Rf 0.26
[ESMS] m/z 313(MH+)

### Example 9: 2-(4-Ethylphenyl)-4-(3-pyridin-2-yl-1H-pyrazol-4-yl)pyridine

A procedure similar to that for Example 1 using Intermediate B9 (800 mg) gave, after precipitation from CH₂Cl₂/hexane, the title compound as a white solid (190 mg).
m.p 96°C
TLC SiO₂ Toluene/isopropylamine 90/10 Rf 0.34
[APCI MS] m/z 327(MH+)

### Example 10: 2-(Thiophen-3-yl)-4-(3-pyridin-2-yl-1H-pyrazol-4-yl)pyridine

A procedure similar to that for Example 1 using Intermediate B10 (1.1 g) gave, after precipitation from ⁱPr₂O, the title compound as an off-white white semi-solid (220 mg).
TLC SiO₂ CH₂Cl₂/MeOH 90/10 Rf 0.55
[APCI MS] m/z 305 (MH+)

### Example 11: 2-[4-(2-Pyrrolidin-1-ylethoxy)-phenyl]-4-(3-pyridin-2-yl-1H-pyrazol-4-yl)pyridine

To a solution of Intermediate E (667 mg, 1.2 mmol) in acetone (20 ml) was added potassium carbonate (4eq, 4.8 mmol, 662 mg) and 1-(2-chloroethyl)pyrrolidine hydrochloride (1.3eq, 1.56mmol, 265mg). The reaction mixture was then heated under reflux overnight and the resulting suspension filtered after cooling. The filtrate was concentrated, dissolved in ethyl acetate and washed with brine. The organic layer was dried over Na₂SO₄, filtered, and evaporated to give the trityl compound as an oil (800 mg). This compound was deprotected without purification. The crude material (800 mg) was dissolved in methanol (30 ml) and HCl (1N, 20 ml) was added. The solution was heated under reflux overnight. The reaction mixture was concentrated in vacuo and the residue dissolved in water and washed with CH₂Cl₂. The aqueous layer was basified with NaOH (1 N) and extracted with EtOAc. The organic extract was washed with water and dried over Na₂SO₄, filtered and evaporated to give a solid which was recrystallised with EtOAc to give the title compound as white crystals (250 mg).
m.p 140°C
[APCI MS] m/z :412 (MH+)

### Example 12:

### 3-({4-[4-(3-Pyridin-2-yl-1H-pyrazol-4-yl)pyridin-2-yl]benzyl}amino)propanenitrile

To a solution of Intermediate F (400 mg, 0.7 mmol) and 3-aminopropionitrile (1.1eq, 54 mg) in dry CH₂Cl₂ was added acetic acid (0.04 ml) followed by sodium triacetoxyborohydride (2eq, 295 mg) and the mixture stirred overnight. After washing with sodium bicarbonate and water, the CH₂Cl₂ solution was treated with TFA (5 ml) and water (5 ml) and stirred overnight. The mixture was neutralised with aq Na₂CO₃ and washed with H₂O. Concentration gave a white solid which was purified by SPE chromatography on silica with CH₂Cl₂:EtOH:NH₃ 200:8:1 to 100 :8:1 as the eluant. This gave the title compound as a white solid (175 mg, 65%).
[APCI MS] m/z: 381 (MH+)
¹H NMR (CDCl₃) 2.5 (2H, t) 2.95 (2H, t), 3.9 (2H, s), 7.28 (2H, m), 7.42 (2H,d) 7.5 (2H, d), 7.63 (1H, dt), 7.8 (2H, d), 7.94 (2H, d) 8.7 (2H, m).

### Example 13: 4-{4-[4-(3-Pyridin-2-yl-1H-pyrazol-4-yl)-pyridin-2-yl]-phenyl}-morpholine

A mixture of Intermediate G (1.1g, 2mmol), di(ethyleneglycol)di-p-tosylate (828mg, 1eq.) and Na₂CO₃ (636 mg, 3eq.) in p-cymene (100ml) was heated at 150°C overnight. The solvent was removed under reduced pressure and the residue dissolved with water. The aqueous phase was extracted with CH₂Cl₂, washed with water and dried over Na₂SO₄ . The solvent was removed *in vacuo* and the crude product was purified by chromatography on silica gel with CH₂Cl₂/MeOH 98:2 to afford the trityl intermediate (200mg). The trityl compound was treated with MeOH / HCl 1N (3:4, 50ml) at reflux for 1h. The reaction mixture was concentrated, basified with NaOH (pH>14) and extracted with CH₂Cl₂. The organic phase was dried over Na₂SO₄ and the solvent removed in vacuo to give a solid which was precipitated with a mixture CH₂Cl₂ / hexane to afford the title compound as a yellow solid (50mg, 6.5%).
m.p 170°C
[APCIMS] m/z 384 (MH+)

### Example 14: 2-[4-(1-Methyl-1-H-imidazol-4-ylmethoxy)-phenyl]-4-(3-pyridin-2-yl-1H-pyrazol-4-yl)-pyridine

Intermediate E and 4-(chloromethyl)-1-methylimidazole hydrochloride were coupled as described for example 11 to afford the title compound as an off-white solid (120mg, 40.2%).
m.p:104°C
[APCIMS] m/z 409 (MH+)

### Example 15: 2-[4-(1-Methyl-1H-imidazol-2-ylmethoxy)-phenyl]-4-(3-pyridin-2-yl-1H-pyrazol-4-yl)pyridine

Intermediate E and 2-(chloromethyl)-1-methylimidazole hydrochloride were coupled as described for example 11 to afford the title compound as a white solid (1.6g, 50%).
m.p:112°C
[APCIMS] m/z 409 (MH+)

### Example 16: 2-[4-(3-Methyl-3H-imidazol-4-ylmethoxy)-phenyl]-4-(3-pyridin-2-yl-1H-pyrazol-4-yl)-pyridine

Intermediate E and 4-(chloromethyl)-3-methylimidazole hydrochloride were coupled as described for example 11 to afford the title compound as a white solid (240mg, 49%).
m.p:130°C
[APCIMS] m/z 409 (MH+).

### Example 17: 2-[4-(2-{1H-Imidazol-1-yl}-ethoxy)-phenyl]-4-(3-pyridin-2-yl-1H-pyrazol-4-yl)-pyridine

Intermediate E and 1-(2-chloroethyl)-imidazole hydrochloride were coupled as described for example 11 to afford the title compound as a white solid (40mg, 16%).
m.p:108°C
[APCIMS] m/z : 409 (MH+)

### Example 18: {4-[4-(3-Pyridin-2-yl-1H-pyrazol-4-yl)pyridin-2-yl]benzyl}-tetrahydro-pyran-4-yl)-amine

To a solution of Intermediate F (24.9g, 45.36mmol) and 4-aminotetrahydropyran (1eq, 4.43g) in dry CH₂Cl₂ (500ml) was added acetic acid (1eq, 2.52 ml) followed by sodium triacetoxyborohydride (2eq, 18.6g). The mixture was stirred overnight at room temperature. After washing with sat. NaHCO₃ (500ml), the aqueous layer was extracted with CH₂Cl₂. The whole organic layer was washed with water and dried over Na₂SO₄ . The solvent was removed under reduced pressure and the resulting residue was treated with a mixture of MeOH/ HCl 1N (3:2, 500ml) at reflux for 2h. The reaction mixture was concentrated, neutralised with sat. NaHCO₃ and washed with H₂O. The organic layer was extracted with CH₂Cl₂ and dried over Na₂SO₄. The solvent was removed in vacuo to give a solid which was purified by chromatography on silica gel with CH₂Cl₂/EtOH 80:20 to afford the title compound as a white solid (8.51g, 45.6%).
m.p:82-85°C.
MS m/z : 412 (MH+)

### Example 19: {4-[4-(3-Pyridin-2-yl-1H-pyrazol-4-yl)pyridin-2-yl]benzyl}-morpholine hydrochloride

To a solution of Intermediate F (1.6g, 2.8mmol) and morpholine (4 eq, 0.97g) in dry dichloroethane (30ml) was added acetic acid (1.5eq, 252mg) followed by sodium triacetoxyborohydride (2eq, 1.2g). The mixture was stirred 10 min at room temperature. The mixture was basified with NaOH 1N, the aqueous layer was extracted with CH₂Cl₂ solution and dried over Na₂SO₄. The solvent was removed under reduced pressure and the resulting product was treated with a mixture of MeOH/ HCl 1N (3:2, 50ml) at reflux for 3h. The reaction mixture was concentrated to dryness, the residue was dissolved in water and washed with CH₂Cl₂. The aqueous layer was basified with NaOH N, extracted with CH₂Cl₂ and dried over Na₂SO₄. Concentration to dryness gave a solid which was purified by chromatography on silica gel with CH₂Cl₂/EtOH 90:10. The resulting product was dissolved in EtOH and a solution of HCl 1N in diethylether (15ml) was added to give after recrystallisation in EtOH the title compound as white crystals (0.7g, 57.6%).
m.p:248°C.
MS m/z: 398 (MH+)

### Example 20: Pyridin-3-yl-methyl-{4-[4-(3-pyridin-2-yl-1H-pyrazol-4-yl)pyridin-2-yl]benzyl}-amine

Intermediate F (800mg, 1.4mmol) and 3-(aminomethyl)pyridine (4eq, 604mg,5.6mmol) were reacted as described for example 19 to afford after precipitation with CH₂Cl₂ / iPr₂O the title compound as a white solid (420mg, 71.8%).
1H-NMR (CDCl3) δ 8.55-8.52 (m, 2H); 8.43 (d, 2H); 8.35 (dd, 1H); 7.79 (d, 2H); 7.64 (d,2H); 7.56 (d, 1H); 7.48 (td, 1H); 7.35-7.27 (m, 3H); 7.15-7.10 (m, 3H); 3.72 (s, 2H); 3.68 (s, 2H).
[APCIMS] m/z: 419.1 (MH+)

### Example 21: 2-Piperidin-1-yl-N-{4-[4-(3-pyridin-2-yl-1H-pyrazol-4-yl)-pyridin-2-yl]-phenyl}-acetamide

To a solution of 1-piperidine acetic acid hydrochloride (233mg, 1.3mmol, 1.3eq.) in CH₂Cl₂ (50ml) were added HOBT (175mg, 1.3eq.) , EDCI (248mg, 1.3eq.) , Et₃N (0.41 ml, 2.3eq.) and Intermediate G (555mg, 1mmol). The reaction mixture was stirred at room temperature overnight. The reaction was hydrolyzed and extracted with CH₂Cl₂. The solvent was removed under reduced pressure. The residue was treated with MeOH/ HCl 1N (3/2, 30ml) at reflux for 1h. After removal of the solvent under reduced pressure, the residue was dissolved into water and washed with CH₂Cl₂. The aqueous phase was basified with NaOH 1N and extracted with CH₂Cl_{2.} The organic phase was dried, filtered, and evaporated to dryness to give a crude solid which was purified by chromatography on silica gel, eluting with toluene/ isopropylamine 90:10. Recrystallisation from EtOH gave the title compound as white crystals (155mg; 35.4%).
m.p: 180°C.
[APCIMS] m/z: 439.2 (MH+).

### Example 22: 2-Pyrrolidin-1-yl-N-{4-[4-(3-pyridin-2-yl-1H-pyrazol-4-yl)-pyridin-2-yl]-phenyl}-acetamide

Intermediate G and 1-pyrrolidineacetic acid were coupled as described for example 21 to afford the title compound as a white solid (90mg, 26.5%).
m.p: 188°C.
[APCIMS] m/z 425 (MH+).

### Example 23: 2-Morpholin-1-yl-N-{4-[4-(3-pyridin-2-yl-1H-pyrazol-4-yl)-pyridin-2-yl]-phenyl}-acetamide

Intermediate G and 1-morpholineacetic acid were coupled as described for example 21 to afford the title compound as a white solid (120mg, 34%).
m.p: 193°C.
[APCIMS] m/z 441 (MH+).

### Example 24: 2-(4-Methyl-piperazin-1-yl)-N-{4-[4-(3-pyridin-2-yl-1H-pyrazol-4-yl)-pyridin-2-yl]-phenyl}-acetamide

Intermediate G and (4-methyl-1-piperazinyl)acetic acid were coupled as described for example 21 to afford the title compound as a white solid (90mg, 24.8%).
m.p: 224°C.
[APCIMS] m/z 454 (MH+).

### Example 25: 3-Piperidin-1-yl-N-{4-[4-(3-pyridin-2-yl-1H-pyrazol-4-yl)-pyridin-2-yl]-phenyl}-propionamide hydrochloride

Intermediate G and 1-piperidinepropanoic acid were coupled as described for example 21. The compound was solubilized in EtOH and treated with a solution of HCl 1N in diethyl ether to afford the title compound as a yellow solid (180mg, 36%).
m.p: 116°C.
[APCIMS] m/z 453 (MH+).

### Example 26: 3-Morpholin-4-yl-N-{4-[4-(3-pyridin-2-yl-1H-pyrazol-4-yl)-pyridin-2-yl]-phenyl}-propionamide

Intermediate G and 3-morpholinepropanoic acid were coupled as described for example 21 to afford the title compound as a white solid (155mg , 48.3%).
m.p: 154°C.
[APCIMS] m/z 455 (MH+).

### Example 27: 3-(4-Methyl-piperazin-1-yl)-N-{4-[4-(3-pyridin-2-yl-1H-pyrazol-4-yl)-pyridin-2-yl]-phenyl}-propionamide

Intermediate G and 3-(4-methyl-piperazine)propanoic acid were coupled as described for example 21 to afford the title compound as a white solid (140mg, 36.5%).
m.p: 232°C.
[APCIMS] m/z 468 (MH+).

### Example 28: 4-[4-(3-Pyridin-2-yl-1H-pyrazol-4-yl)-pyridin-2-yl]-N-(tetrahydro-pyran-4-yl)-benzamide

Intermediate J and 4-aminotetrahydropyran were reacted as described for example 21 to afford the title compound as a white solid (200mg, 55%).
m.p: 248°C.
[APCIMS] m/z 426 (MH+).

### Example 29: N-(1-Ethyl-pyrolidin-2-ylmethyl]-4-[4-(3-pyridin-2-yl-1H-pyrazol-4-yl)-pyridin-2-yl]-benzamide

Intermediate J and 2-aminomethyl-1-ethylpyrrolidine were coupled as described for example 21 to afford the title compound as an off-white powder (58mg, 23%).
m.p: 96°C.
[APCIMS] m/z: 453 (MH+).

### Example 30: (1-Ethyl)-{4-[4-(3-pyridin-2-yl-1H-pyrazol-4-yl)pyridin-2-yl]-phenyl}-piperazine

A mixture of intermediate K1 (867 mg, 1.4 mmole), *N*-ethylpiperazine (4 eq, 0,71 ml, 5.6 mmol), Pd₂(dba)₃ (0.05 eq, 64 mg, 0.07 mmol), BINAP (0.1 eq, 91 mg, 0.14 mmol) and sodium *tert*-butoxide (1.4 eq, 188 mg, 1.96 mmol) in toluene (50 ml) was heated under reflux for 18 h. After dilution with toluene, the organic phase was washed with water, then brine, and dried (Na₂SO₄). The solvent was removed under reduced pressure and the resulting residue purified by chromatography on silicagel eluting with CH₂Cl₂:MeOH (97:3). The resulting residue was treated with a mixture of MeOH/HCl 1N (3:2, 30 ml) at reflux during 1 h. The reaction mixture was concentrated, neutralised with a sat. NaHCO₃ solution and extracted with CH₂Cl₂. The organic phase was dried (Na₂SO₄), and concentrated under reduced pressure. The resulting residue was purified by chromatography on silicagel, eluting with CH₂Cl₂: MeOH (95/5). After crystallisation from EtoAc, the title compound was obtained as a white solid (67 mg, 11.7%).
m.p: 194°C
[APCIMS] m/z 411 (MH+)

### Example 31: (N-Methyl)-({4-[4-(3-pyridin-2-yl-1H-pyrazol-4-yl)pyridin-2-yl]-phenyl}-tetrahydropyran-4-yl)-amine

Intermediate K1 ( 929 mg, 1.5 mmol) and 4-(*N*-methylamino)-tetrahydropyran, hydrochloride (1.5 eq, 341 mg, 2.25 mmol) were reacted as described for example 30 to afford, after precipitation with hexane, the title compound as a yellow solid (44 mg, 7%), gummy at 132°C.
[APCIMS] m/z 411 (MH+)

### Example 32: ({4-[4-(3-Pyridin-2-yl-1H-pyrazol-4-yl)pyridin-2-yl]-phenyl}-tetrahydropyran-4-yl)-amine

Intermediate K1 (1.1 g, 177 mmol) and 4-amino-tetrahydropyran (2 eq, 358 mg, 3.54 mmol) were reacted as described for example 30 to afford after Precipitation with hexane, the title compound as a yellow solid (129 mg, 18%), gummy at 140°C.
[APCIMS] m/z 398 (MH+)

### Example 33: N-Methyl-({4-[4-(3-pyridin-2-yl-1H-pyrazol-4-yl)pyridin-2-yl]benzyl}-tetrahydro-pyran-4-yl)-amine

Intermediate F (900 mg, 1.58 mmol) and *N*-methyl-(tetrahydropyran-4-yl)-amine (2 eq, 364 mg, 3.16mmol) were reacted as described for example 19 to afford after precipitation from hexane, the title compound as a white solid (138 mg, 20%), gummy at 100°C.
[APCIMS] m/z 426 (MH+).

### Example 34: 4-Methoxy-1-{4-[4-(3-pyridin-2-yl-1H-pyrazol-4-yl)pyridin-2-yl]benzyl}-piperidine

Intermediate F (1 g, 1.76 mmol) and 4-methoxy-piperidine hydrochloride (1 eq, 267 mg, 1.76 mmol ) were reacted as described for example 19 to afford after precipitation from hexane, the title compound as a grey solid (195 mg, 26%), gummy at 100°C.
[APCIMS] m/z 426 (MH+)

### Example 35: {1-[4-(3-Pyridin-2-yl-1H-pyrazol-4-yl)pyridin-2-yl]benzyl}-pyrrolidine

Intermediate F (1 g, 1.76 mmol) and pyrrolidine (1.1 eq, 0.16 ml, 1.94 mmol) were reacted as described for example 19 to afford the title compound as a white gummy solid (170 mg, 25.3 %).
[APCIMS] m/z 382 (MH+).

### Example 36: (1-Methyl)-{4-[4-(3-pyridin-2-yl-1H-pyrazol-4-yl)pyridin-2-yl]benzyl}-piperazine

Intermediate F (1 g, 1.76 mmol) and *N*-methyl-piperazine (1.1 eq, 0.21 ml, 1.94 mmol) were reacted as described for example 19 to afford after precipitation from pentane, the title compound as a white solid (300 mg, 41.5 %).
m.p.: 215-217°C
[APCIMS] m/z 411 (MH+).

### Example 37: (1-Methyl-piperidin-4-yl)-{4-[4-(3-pyridin-2-yl-1H-pyrazol-4-yl)pyridin-2-yl]benzyl}-amine

Intermediate F (700 mg, 1.26 mmol) and 4-amino-1-methyl-piperidine (1.1 eq, 150 mg, 1.38 mmol ) were reacted as described for example 19 to afford after precipitation from pentane, the title compound as a cream solid (150 mg, 28.7 %).
m.p.: 150-155°C
[APCIMS] m/z 425 (MH+).

### Example 38: 1-Methyl-4-{4-[4-(3-pyridin-2-yl-1H-pyrazol-4-yl)-pyridin-2-yl]-benzoyl}-piperazine

Intermediate J (584 mg, 1 mmol) and 1-methyl-piperazine (2 eq, 200 mg, 2 mmol) were coupled as described for example 21 to afford the title compound as a white solid (100 mg, 23.5 %).
m.p: 142°C.
[APCIMS] m/z 425 (MH+).

### Example 39: 4-{4-[4-(3-Pyridin-2-yl-1H-pyrazol-4-yl)-pyridin-2-yl]-benzoyl}-morpholine

Intermediate J (584 mg, 1 mmol) and morpholine (2 eq, 180 mg, 2 mmol) were coupled as described for example 21 to afford the title compound as a white solid (300 mg, 72.8 %).
m.p: 155°C.
[APCIMS] m/z 412 (MH+).

### Example 40: (4-Dimethylamino)-1-{4-[4-(3-pyridin-2-yl-1H-pyrazol-4-yl)-pyridin-2-yl]-benzoyl}-piperidine

Intermediate J (584 mg, 1 mmol) and 4-dimethylamino-piperidine hydrochloride (2 eq, 402 mg, 2 mmol) were coupled as described for example 21 to afford the title compound as a white solid (90 mg, 19.9 %).
m.p: 185°C.
[APCIMS] m/z 453 (MH+).

### Example 41: (1-Methyl)-{4-[4-(3-pyridin-2-yl-1H-pyrazol-4-yl)pyridin-2-yl]-phenyl}-piperazine

A mixture of Intermediate G (1.1g, 2mmol), *N*-methylbis-(2-chloroethyl)-amine hydrochloride (1eq, 294mg, 2mmol) and Na₂CO₃ (636 mg, 3eq.) in butanol (50ml) was refluxed overnight. To complete the reaction *N*-methylbis-(2-chloroethyl)-amine hydrochloride (1eq, 294mg) was added and the reaction mixture was refluxed for 48h. The solvent was removed under reduced pressure and the residue dissolved with water. The aqueous phase was extracted with CH₂Cl₂, washed with water and dried over Na₂SO₄ The solvent was removed *in vacuo* and the crude product was purified by chromatography on silica gel with CH₂Cl₂/MeOH 95:5 to afford the trityl intermediate (200mg). The trityl compound was treated with MeOH / HCl 1N (3:4, 20ml) at reflux for 1h. The reaction mixture was concentrated, basified with NaOH (pH>14) and extracted with CH₂Cl₂. The organic phase was dried over Na₂SO₄, and concentrated under reduced pressure to give a solid which was precipitated with a mixture CH₂Cl₂ / diisopropyl ether to afford the title compound as a yellow solid (90mg, 11.4%).
m.p: 189°C
[APCIMS] m/z 397 (MH+).

### Example 42: {4-[4-(3-Pyridin-2-yl-1H-pyrazol-4-yl)pyridin-2-yl]benzyl}-thiomorpholine

Intermediate F (711 mg, 1.25mmol) and thiomorpholine (2 eq, 258 mg, 2.5mmol) were reacted as described for example 19 to afford after precipitation from hexane, the title compound as a white solid (250 mg, 48%), gummy at 80°C.
¹H NMR (300 MHz, CDCl₃) δ: 8.62 (m, 2H); 7.85 (d, 2H); 7.72 (m,2H); 7.57 (td, 1H); 7.42 (m, 1H); 3.5 (s, 2H); 2. (m, 8H), NH not observed.
[APCIMS] m/z 426 (MH+)

### Example 43: Dimethyl-{4-[4-(3-pyridin-2-yl-1H-pyrazol-4-yl)pyridin-2-yl]-benzyl}-amine

Intermediate F (1g, 1.80mmol) and dimethylamine hydrochloride (1.1 eq, 161mg, 1.97mmol) were reacted as described for example 19 to afford after precipitation from hexane, the title compound as a pale pink powder (500 mg, 78%).
¹H NMR (300 MHz, CDCl₃) δ: 8.6 (m, 2H); 7.85 (d, 2H); 7.7 (m, 2H); 7.55 (m, 1H); 7.4 (m,1H); 7.3 (m, 2H); 7.2 (m, 2H); 3.4 (s, 2H); 3.3 ( m, 4H); 2.2 (s, 6H); 1.6 (m, 2H), NH not observed.

### Example 44: 4-[4-(3-Pyridin-2-yl-1H-pyrazol-4-yl)-pyridin-2-yl]-N-(tetrahydropyran-4-yl-methyl)-benzamide

Intermediate J (584mg, 1mmol) and 4-aminomethyltetrahydropyran (130mg, 1.1mmol) were coupled as described for example 21 to afford the title compound as a colourless oil (400mg, 91%).
¹H NMR (300 MHz, CDCl₃) δ: 8.6 (m, 2H); 7.9 (d, 2H); 7.8 (m, 2H); 7.7 (s, 1H); 7.55 (m, 1H); 7.4 (m,1H); 7.3 (m, 1H); 7.2 (m, 2H); 6.3 (m, 1H); 3.9 (m, 2H); 3.3 ( m, 4H); 1.8 (m, 1H); 1.6 (m, 2H); 1.3 (m, 2H).
[APCIMS] m/z 440.17 (MH+).

### Example 45: N-(2-Methoxy-ethyl)-N-methyl-4-[4-(3-pyridin-2-yl-1H-pyrazol-4-yl)-pyridin-2-yl]-benzamide

Intermediate J (1g, 1.7mmol) and 2-methoxy-*N*-methylethylamine (175mg, 2mmol) were coupled as described for example 21 to afford the title compound as a colourless oil.
¹H NMR (300 MHz, CDCl₃) δ: 8.6 (m, 2H); 7.9 (m, 2H); 7.7 (m, 2H); 7.6 (m, 1H); 7.4 (m,3H); 7.2 (m, 2H); 3.6 (m, 3H); 3.5-2.9 (m, 7H); NH not observed. [APCIMS] m/z 414.03 (MH+)

### Example 46: N-(2-Methoxy-ethyl)-4-[4-(3-pyridin-2-yl-1H-pyrazol-4-yl)-pyridin-2-yl]-benzamide

Intermediate J (400mg, 0.7mmol) and 2-methoxyethylamine (55.6mg, 0.7mmol) were coupled and treated as described for example 21 to afford the title compound as white crystals (104mg, 37%).
m.p 95°C
[APCIMS] m/z 400 (MH+)

### Example 47: N-Cyclohexylmethyl-4-[4-(3-pyridin-2-yl-1H-pyrazol-4-yl)-pyridin-2-yl]-benzamide

Intermediate J (400mg, 0.7mmol) and cyclohexylmethylamine (79.3mg, 0.7mmol) were coupled and treated as described for example 21 to afford the title compound as white crystals (111mg, 36%).
m.p 120°C
[APCIMS] m/z 438 (MH+)

### Biological Data

The compounds of Examples 1-47 were tested *in vitro,* using the biological assays described below. All of the compounds had an IC₅₀ value of 5 µM or below in Assay 1, and an IC₅₀ value of 1 µM or below in Assay 2.

### Assays

### Assay 1

The potential for compounds of the invention to inhibit TGF- β signaling may be demonstrated, for example, using the following *in vitro* assay.

The assay was performed in HepG2 cells stably transfected with the PAI-1 promoter (known to be a strong TGF-β responsive promoter) linked to a luciferase (firefly) reporter gene. The compounds were selected on their ability to inhibit luciferase activity in cells exposed to TGF-β. In addition cells were transfected with a second luciferase (Renilla) gene which was not driven by a TGF-β responsive promoter and was used as a toxicity control.

(96 well-)microplates are seeded, using a multidrop apparatus, with the stably transfected cell line at a concentration of 35000 cells per well in 200 µl of serum-containing medium. These plates are placed in a cell incubator.

18 to 24 hours later (Day 2), cell-incubation procedure is launched. Cells are incubated with TGF-β and a candidate compound at concentrations in the range 50 nM to 10 µM (final concentration of DMSO 1 %). The final concentration of TGF-β (rhTGFβ-1) used in the test is 1 ng/mL. Cells are incubated with a candidate compound 15-30 mins prior to the addition of TGF-β. The final volume of the test reaction is 150 µl. Each well contains only one candidate compound and its effect on the PAI-1 promoter is monitored.

Columns 11 and 12 are employed as controls. Column 11 contains 8 wells in which the cells are incubated in the presence of TGF-β, *without* a candidate compound. Column 11 is used to determine the 'reference TGF-β induced firefly luciferase value' against which values measured in the test wells (to quantify inhibitory activity) may be compared. In wells A12 to D12, cells are grown in medium without TGF-β. The firefly luciferase values obtained from these positions are representive of the 'basal firefly luciferase activity'. In wells E12 to H12, cells are incubated in the presence of TGF-β and 500 µM CPO (Cyclopentenone, Sigma), a cell toxic compound. The toxicity is revealed by decreased firefly and renilla luciferase activities (around 50 % of those obtained in column 11).

12 to 18 hours later (day 3), the luciferase quantification procedure is launched. The following reactions are performed using reagents obtained from a Dual Luciferase Assay Kit (Promega). Cells are washed and lysed with the addition of 10 µl of passive lysis buffer (Promega). Following agitation (15 to 30 mins), luciferase activities of the plates are read in a dual-injector luminometer (BMG lumistar). For this purpose, 50 µl of luciferase assay reagent and 50 µl of 'Stop & Glo' buffer are injected sequentially to quantify the activities of both luciferases. Data obtained from the measurements are processed and analysed using suitable software. The mean Luciferase activity value obtained in wells A11 to H11 (Column 11, TGF-β only) is considered to represent 100% and values obtained in wells A12 to D12 (cells in medium alone) give a basal level (0%).

For each of the compounds tested, a concentration response curve is constructed from which an IC₅₀ value can be determined graphically.

### Assay 2

The potential for compounds of the invention to inhibit the kinase Alk5 receptor may be demonstrated, for example, using the following *in vitro* assay.

The kinase domain of Alk5 was cloned and expressed in a baculovirus/Sf9 cells system. The protein (amino acids 162 to 503) was 6-His tagged in C-terminus. After purification by affinity chromatography using a Ni²⁺ column, the autophosphorylation was tested. The enzyme was incubated in a medium containing : Tris 50 mM pH 7.4; NaCl 100 mM ; MgCl₂ 5 mM ; MnCl₂ 5 mM ; DTT 10 mM. The enzyme was preincubated with the compounds (0.1% DMSO final in the test) 10 minutes at 37°C. The reaction was initialised by the addition of 3 µM ATP (0.5 µCi gamma-33P-ATP). After 15 minutes at 37°C the reaction was stopped by addition of SDS-PAGE sample buffer (50 mM Tris-HCl, pH 6.9, 2.5 % glycerol, 1% SDS, 5 % beta-mercaptoethanol). The samples were boiled for 5 minutes at 95°C and run on a 12% SDS-PAGE. The dried gels were exposed to a phosphor screen over-night. Alk5 autophosphorylation was quantified using a STORM (Molecular Dynamics).

## Claims

1. A compound of formula (I), 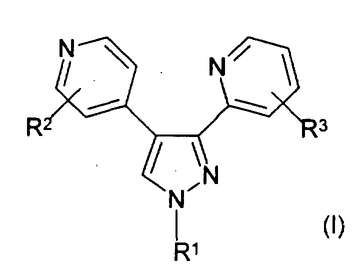 wherein,
R¹ is selected from H, C₁₋₄ alkyl or CH₂CONR⁴R⁵, wherein R⁴ is selected from H or C₁₋₄ alkyl and R⁵ is C₁₋₄ alkyl;
R² is selected from phenyl, furanyl or thiophenyl, wherein the phenyl may be further substituted by one or more substituents, which may be the same or different, selected from halo (such as fluoro, chloro, bromo), -CN, -CF₃, -OCF₃, C₁₋₄ alkyl, -OR⁶, -O-(CH₂)ₙ-XR⁶R⁷, -O-(CH₂)ₙ-OR⁶, -O-(CH₂)ₙ-COR⁶, -O-(CH₂)ₙ-C₂-₆ alkenyl, -O-(CH₂)ₙ-C₂₋₆ alkynyl, -(CH₂)ₙ-NR⁶R⁷, -CONR⁶R⁷, -NHCOR⁶ and -NR⁶R⁷ where n is an integer value from 1 to 6 and where X is an atom selected from C, N or S, and wherein the furanyl and thiophenyl may be further substituted by one or more substituents, which may be the same or different, selected from halo (such as fluoro, chloro, bromo), -CN, -CF₃, -OH, -OCF₃, C₁₋₄ alkyl and C₁₋₄ alkoxy;
R⁶ and R⁷ which may be the same or different, are selected from H, C₁₋₆ alkyl, cycloalkyl, cycloalkyl C₁₋₆ alkyl, aryl, arylC₁₋₆ alkyl, heteroaryl, heteroarylC₁₋₆ alkyl, heterocyclyl, heterocyclylC₁₋₆ alkyl, C₁₋₄ alkoxyC₁₋₆ alkyl, hydroxyC₁₋₆ alkyl, -(CH₂)ₙ-NR⁸R⁹, or R⁶ and R⁷ together with the atom to which they are attached form a 3, 4, 5, 6 or 7 membered saturated or unsaturated ring which may contain one or more heteroatoms selected from N, S or O, and wherein the ring may be further substituted by one or more substituents selected from halo (such as fluoro, chloro, bromo), -CN, -CF₃, -OH, -OCF₃, C₁₋₄ alkyl, C₁₋₄ alkoxy and NR⁸R⁹;
R⁸ and R⁹ which may be the same or different are selected from H or C₁₋₆ alkyl, wherein the C₁₋₆ alkyl may be further substituted one or more substituents selected from halo, -CN, -CF₃, and -OH;
R³ is selected from H, halo, -CN, -CF₃, C₁₋₄ alkyl or C₁₋₄ alkoxy;
and salts and solvates thereof.

2. A compound of formula (I) as claimed in claim 1, wherein R¹ is H or C₁₋₄ alkyl.

3. A compound of formula (I) as claimed in either claim 1 or claim 2, wherein R² is located at the C(2) position of the pyridinyl ring and represents phenyl which may be further substituted by one or more substituents as defined in claim 1.

4. A compound of formula (I) as claimed in claim 3, wherein R² is located at the C(2) position of the pyridinyl ring and represents phenyl which may be further substituted at the para position by one or more substituents as defined in claim 1.

5. A compound of formula (I) as claimed in any one of claims 1 to 4, wherein R² is located at the C(2) position of the pyridinyl ring and represents thiophenyl which may be further substituted by one or more substituents as defined in claim 1.

6. A compound of formula (I) as claimed in any one of the preceding claims, wherein R³ is located at the C(3) or C(6) position of the pyridine ring and is selected from H, halo, -CN, -CF₃, C₁₋₄ alkyl or C₁₋₄ alkoxy.

7. A compound of formula (I) as claimed in claim 1 selected from:
2-Phenyl-4-(3-pyridin-2-yl-1H-pyrazol-4-yl)pyridine;
2-[4-(Trifluoromethyl)phenyl]-4-(3-pyridin-2-yl-1H-pyrazol-4-yl)pyridine;
2-(4-Methoxyphenyl)-4-(3-pyridin-2-yl-1H-pyrazol-4-yl)pyridine;
2-(4-Fluorophenyl)-4-(3-pyridin-2-yl-1H-pyrazol-4-yl)pyridine;
2-(4-Chlorophenyl)-4-(3-pyridin-2-yl-1H-pyrazol-4-yl)pyridine;
2-(Furan-2-yl)-4-(3-pyridin-2-yl-1H-pyrazol-4-yl)pyridine;
2-(4-Trifluoromethoxyphenyl)-4-(3-pyridin-2-yl-1H-pyrazol-4-yl)pyridine;
2-(4-Methylphenyl)-4-(3-pyridin-2-yl-1H-pyrazol-4-yl)pyridine;
2-(4-Ethylphenyl)-4-(3-pyridin-2-yl-1H-pyrazol-4-yl)pyridine;
2-(Thiophen-3-yl)-4-(3-pyridin-2-yl-1H-pyrazol-4-yl)pyridine;
2-[4-(1-Methyl-1H-imidazol-2-ylmethoxy)-phenyl]-4-(3-pyridin-2-yl-1H-pyrazol-4-yl)pyridine;
2-[4-(3-Methyl-3H-imidazol-4-ylmethoxy)-phenyl]-4-(3-pyridin-2-yl-1H-pyrazol-4-yl)-pyridine;
2-[4-(2-{1H-Imidazol-1-yl}-ethoxy)-phenyl]-4-(3-pyridin-2-yl-1H-pyrazol-4-yl)pyridine;
2-[4-(2-Pyrrolidin-1-ylethoxy)-phenyl]-4-(3-pyridin-2-yl-1H-pyrazol-4-yl)pyridine;
3-({4-[4-(3-Pyridin-2-yl-1*H*-pyrazol-4-yl)pyridin-2-yl]benzyl}amino)propanenitrile;
4-{4-[4-(3-Pyridin-2-yl-1H-pyrazol-4-yl)-pyridin-2-yl]-phenyl}-morpholine;
2-[4-(1-Methyl-1-H-imidazol-4-ylmethoxy)-phenyl]-4-(3-pyridin-2-yl-1H-pyrazol-4-yl)-pyridine;
({4-[4-(3-Pyridin-2-yl-1H-pyrazol-4-yl)pyridin-2-yl]benzyl}-tetrahydro-pyran-4-yl)-amine;
{4-[4-(3-Pyridin-2-yl-1H-pyrazol-4-yl)pyridin-2-yl]benzyl}-morpholine hydrochloride;
Pyridin-3-ylmethyl-{4-[4-(3-pyridin-2-yl-1H-pyrazol-4-yl)pyridin-2-yl]benzyl}-amine;
2-Piperidin-1-yl-N-{4-[4-(3-pyridin-2-yl-1H-pyrazol-4-yl)-pyridin-2-yl]-phenyl}-acetamide;
2-Pyrrolidin-1-yl-N-{4-[4-(3-pyridin-2-yl-1H-pyrazol-4-yl)-pyridin-2-yl]-phenyl}-acetamide;
2-Morpholin-1-yl-N-{4-[4-(3-pyridin-2-yl-1H-pyrazol-4-yl)-pyridin-2-yl]-phenyl}-acetamide;
2-(4-Methyl-piperazin-1-yl)-N-{4-[4-(3-pyridin-2-yl-1H-pyrazol-4-yl)-pyridin-2-yl]-phenyl}-acetamide;
3-Piperidin-1-yl-N-{4-(4-(3-pyridin-2-yl-1H-pyrazol-4-yl)-pyridin-2-yl)-phenyl}-propionamide hydrochloride;
3-Morpholin-4-yl-N-{4-[4-(3-pyridin-2-yl-1H-pyrazol-4-yl)-pyridin-2-yl]-phenyl}-propionamide;
3-(4-Methyl-piperazin-1-yl)-N-{4-[4-(3-pyridin-2-yl-1H-pyrazol-4-yl)-pyridin-2-yl]-phenyl}-propionamide;
4-[4-(3-Pyridin-2-yl-1H-pyrazol-4-yl)-pyridin-2-yl]-N-(tetrahydro-pyran-4-yl)-benzamide;
N-(1-Ethyl-pyrrolidin-2-ylmethyl)4-[4-(3-pyridin-2-yl-1H-pyrazol-4-yl)-pyridin-2-yl]-benzamide;
(1-Ethyl)-{4-[4-(3-pyridin-2-yl-1*H*-pyrazol-4-yl)pyridin-2-yl]-phenyl}-piperazine; (*N*-Methyl)-({4-(4-(3-pyridin-2-yl-1*H*-pyrazol-4-yl)pyridin-2-yl]-phenyl}-tetrahydro-pyran-4-yl)-amine;
({4-[4-(3-Pyridin-2-yl-1*H*-pyrazol-4-yl)pyridin-2-yl]-phenyl}-tetrahydro-pyran-4-yl)-amine;
*N*-Methyl-({4-[4-(3-pyridin-2-yl-1*H*-pyrazol-4-yl)pyridin-2-yl]benzyl}-tetrahydro-pyran-4-yl)-amine;
4-Methoxy-1-{4-[4-(3-pyridin-2-yl-1*H*-pyrazol-4-yl)pyridin-2-yl]benzyl}-piperidine;
{1-[4-(3-Pyridin-2-yl-1*H*-pyrazol-4-yl)pyridin-2-yl]benzyl}-pyrrolidine;
(1-Methyl)-{4-[4-(3-pyridin-2-yl-1*H*-pyrazol-4-yl)pyridin-2-yl]benzyl}-piperazine;
(1-Methyl-piperidin-4-yl)-{4-[4-(3-pyridin-2-yl-1*H*-pyrazol-4-yl)pyridin-2-yl]benzyl}-amine;
1-Methyl-4-{4-[4-(3-pyridin-2-yl-1*H*-pyrazol-4-yl)-pyridin-2-yl]-benzoyl}-piperazine;
4-{4-[4-(3-Pyridin-2-yl-1*H*-pyrazol-4-yl)-pyridin-2-yl]-benzoyl}-morpholine;
(4-Dimethylamino)-1-{4-[4-(3-pyridin-2-yl-1*H*-pyrazol-4-yl)-pyridin-2-yl]-benzoyl}-piperidine;
(1-Methyl)-{4-[4-(3-pyridin-2-yl-1*H*-pyrazol-4-yl)pyridin-2-yl]-phenyl}-piperazine;
{4-[4-(3-Pyridin-2-yl-1H-pyrazol-4-yl)pyridin-2-yl]benzyl}-thiomorpholine;
Dimethyl-{4-[4-(3-pyridin-2-yl-1*H*-pyrazol-4-yl)pyridin-2-yl]-benzyl}-amine;
4-[4-(3-Pyridin-2-yl-1H-pyrazol-4-yl)-pyridin-2-yl]-N-(tetrahydro-pyran-4-yl-methyl)-benzamide;
N-(2-Methoxy-ethyl)-*N*-methyl-4-[4-(3-pyridin-2-yl-1H-pyrazol-4-yl)-pyridin-2-yl]-benzamide;
N-(2-Methoxy-ethyl)-4-[4-(3-pyridin-2-yl-1H-pyrazol-4-yl)-pyridin-2-yl]-benzamide; and
N-Cyclohexylmethyl-4-[4-(3-pyridin-2-yl-1H-pyrazol-4-yl)-pyridin-2-yl]-benzamide;
and salts and solvates thereof.

8. A pharmaceutical composition comprising a compound of formula (I) as claimed in any one of claims 1 to 7, together with a pharmaceutically acceptable diluent or carrier.

9. A compound of formula (I) as claimed in any one of claims 1 to 7, for use as a medicament.

10. The use of a compound of formula (I) as claimed in any one of claims 1 to 7 in the manufacture of a medicament for the treatment or prophylaxis of a disorder **characterised by** the over expression of TGF-β.

11. The use of a compound of formula (I) as claimed in claim 10, wherein the disorder is selected from fibrosis, especially liver and kidney fibrosis, cancer development, abnormal bone function and inflammatory disorders and scarring.

12. The use of a compound of formula (I) as claimed in claim 10 or claim 11 in combination with an antiviral agent, or in combination with ACE inhibitors or Angiotensin II receptor antagonists.

13. A process for the preparation of a compound of formula (I), 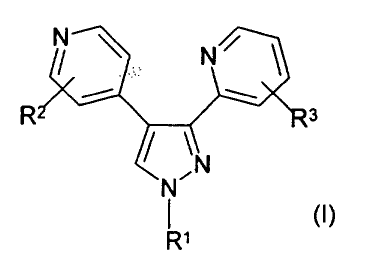 wherein,
R¹ is H;
R² is selected from phenyl, furanyl or thiophenyl, wherein the phenyl may be further substituted by one or more substituents, which may be the same or different, selected from halo (such as fluoro, chloro, bromo), -CN, -CF₃, -OCF₃, C₁₋₄ alkyl, -OR⁶, -O-(CH₂)ₙ-XR⁶R⁷, -O-(CH₂)ₙ-OR⁶, -O-(CH₂)ₙ-COR⁶, -O-(CH₂)ₙ-C₂-₆ alkenyl, -O-(CH₂)ₙ-C₂-₆ alkynyl, -(CH₂)ₙ-NR⁶R⁷, -CONR⁶R⁷, -NHCOR⁶ and -NR⁶R⁷ where n is an integer value from 1 to 6 and where X is an atom selected from C, N or S, and wherein the furanyl and thiophenyl may be further substituted by one or more substituents, which may be the same or different, selected from halo (such as fluoro, chloro, bromo), -CN, -CF₃, -OH, -OCF₃, C₁₋₄ alkyl and C₁₋₄ alkoxy;
R⁶ and R⁷ which may be the same or different, are selected from H, C₁₋₆ alkyl, cycloalkyl, cycloalkyl C₁₋₆ alkyl, aryl, arylC₁₋₆ alkyl, heteroaryl, heteroarylC₁₋₆ alkyl, heterocyclyl, heterocyclylC₁₋₆ alkyl, C₁₋₄ alkoxyC₁₋₆ alkyl, hydroxyC₁₋₆ alkyl, -(CH₂)ₙ-NR⁸R⁹, or R⁶ and R⁷ together with the atom to which they are attached form a 3, 4, 5, 6 or 7 membered saturated or unsaturated ring which may contain one or more heteroatoms selected from N, S or O, and wherein the ring may be further substituted by one or more substituents selected from halo (such as fluoro, chloro, bromo), -CN, -CF₃, -OH, -OCF₃, C₁₋₄ alkyl, C₁₋₄ alkoxy and NR⁸R⁹;
R⁸ and R⁹ which may be the same or different are selected from H or C₁₋₆ alkyl, wherein the C₁₋₆ alkyl may be further substituted one or more substituents selected from halo, -CN, -CF₃, and -OH;
R³ is selected from H, halo, -CN, -CF₃, C₁₋₄ alkyl or C₁₋₄ alkoxy;
which process comprises:
a) addition of dimethylformamide dimethylacetal to a ketone of formula (B) 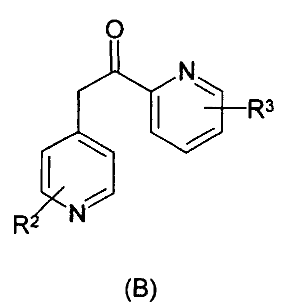 wherein R² and R³ are as defined above; and
b) subsequent addition of hydrazine monohydrate, NH₂NH₂.H₂O to the resulting reaction mixture.

14. A process for the preparation of a compound of formula (I), 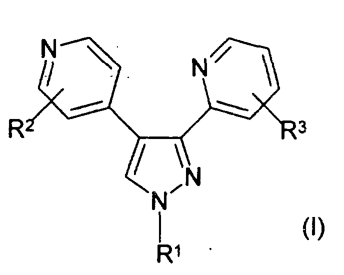 wherein,
R¹ is C₁₋₄ alkyl or CH₂CONR⁴R⁵, wherein R⁴ is selected from H or C₁₋₄ alkyl and R⁵ is C₁₋₄ alkyl;
R² is selected from phenyl, furanyl or thiophenyl, wherein the phenyl may be further substituted by one or more substituents, which may be the same or different, selected from halo (such as fluoro, chloro, bromo), -CN, -CF₃, -OCF₃, C₁₋₄ alkyl, -OR⁶, -O-(CH₂)ₙ-XR⁶R⁷, -O-(CH₂)ₙ-OR⁶, -O-(CH₂)ₙ-COR⁶, -O-(CH₂)ₙ-C₂-₆ alkenyl, -O-(CH₂)ₙ-C₂-₆ alkynyl, -(CH₂)ₙ-NR⁶R⁷, -CONR⁶R⁷, -NHCOR⁶ and -NR⁶R⁷ where n is an integer value from 1 to 6 and where X is an atom selected from C, N or S, and wherein the furanyl and thiophenyl may be further substituted by one or more substituents, which may be the same or different, selected from halo (such as fluoro, chloro, bromo), -CN, -CF₃, -OH, -OCF₃, C₁₋₄ alkyl and C₁₋₄ alkoxy;
R⁶ and R⁷ which may be the same or different, are selected from H, C₁₋₆ alkyl, cycloalkyl, cycloalkyl C₁₋₆ alkyl, aryl, arylC₁₋₆ alkyl, heteroaryl, heteroarylC₁₋₆ alkyl, heterocyclyl, heterocyclylC₁₋₆ alkyl, C₁₋₄ alkoxyC₁₋₆ alkyl, hydroxyC₁₋₆ alkyl, -(CH₂)ₙ-NR⁸R⁹, or R⁶ and R⁷ together with the atom to which they are attached form a 3, 4, 5, 6 or 7 membered saturated or unsaturated ring which may contain one or more heteroatoms selected from N, S or O, and wherein the ring may be further substituted by one or more substituents selected from halo (such as fluoro, chloro, bromo), -CN, -CF₃, -OH, -OCF₃, C₁₋₄ alkyl, C₁₋₄ alkoxy and NR⁸R⁹;
R⁸ and R⁹ which may be the same or different are selected from H or C₁₋₆ alkyl, wherein the C₁₋₆ alkyl may be further substituted one or more substituents selected from, halo, -CN, -CF₃, and -OH;
R³ is selected from H, halo, -CN, -CF₃, C₁₋₄ alkyl or C₁₋₄ alkoxy;
which process comprises N-alkylation of a compound of formula (I) where R¹ is H, in the presence of a suitable base.

## Patentansprüche

1. Verbindung der Formel (I), 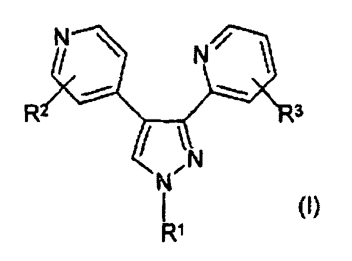 wobei
R¹ ausgewählt ist aus H, einem C₁₋₄-Alkylrest oder CH₂CONR⁴R⁵, wobei R⁴ ausgewählt ist aus H oder einem C₁₋₄-Alkylrest und R⁵ ein C₁₋₄-Alkylrest ist;
R² ausgewählt ist aus einer Phenyl-, Furanyl- oder Thiophenylgruppe, wobei die Phenylgruppe ferner mit einem oder mehreren Substituenten substituiert sein kann, welche gleich oder verschieden sein können, ausgewählt aus einem Halogenatom (wie Fluor, Chlor, Brom), -CN, -CF₃, -OCF₃, einem C₁₋₄-Alkylrest, -OR⁶, -O-(CH₂)ₙ-XR⁶R⁷, -O-(CH₂)ₙ-OR⁶, -O-(CH₂)ₙ-COR⁶, -O-(CH₂)ₙ-C₂₋₆-Alkenyl, -O-(CH₂)ₙ-C₂₋₆-Alkinyl, -(CH₂)ₙ-NR⁶R⁷, -CONR⁶R⁷, -NHCOR⁶ und -NR⁶R⁷, wobei n eine ganze Zahl mit einem Wert von 1 bis 6 ist und wobei X ein Atom ist, ausgewählt aus C, N oder S, und wobei die Furanyl- und Thiophenylgruppe ferner mit einem oder mehreren Substituenten substituiert sein können, welche gleich oder verschieden sein können, ausgewählt aus einem Halogenatom (wie Fluor, Chlor, Brom), -CN, -CF₃, -OH, -OCF₃, einem C₁₋₄-Alkylrest und einem C₁₋₄-Alkoxyrest;
R⁶ und R⁷, welche gleich oder verschieden sein können, ausgewählt sind aus H, einem C₁₋₆-Alkyl-, Cycloalkyl-, Cycloalkyl-C₁₋₆-alkyl-, Aryl-, Aryl-C₁₋₆-alkyl-, Heteroaryl-, Heteroaryl-C₁₋₆-alkyl-, Heterocyclyl-, Heterocyclyl-C₁₋₆-alkyl-, C₁₋₄-Alkoxy-C₁₋₆-alkyl-, Hydroxy-C₁₋₆-alkylrest, -CH₂)ₙ-NR⁸R⁹, oder R⁶ und R⁷ zusammen mit dem Atom, an das sie gebunden sind, einen 3-, 4-, 5-, 6- oder 7-gliedrigen gesättigten oder ungesättigten Ring bilden, welcher ein oder mehrere aus N, S oder O ausgewählte Heteroatome enthalten kann und wobei der Ring ferner mit einem oder mehreren Substituenten substituiert sein kann, ausgewählt aus einem Halogenatom (wie Fluor, Chlor, Brom), -CN, -CF₃, -OH, -OCF₃, einem C₁₋₄-Alkylrest, einem C₁₋₄-Alkoxyrest und NR⁸R⁹;
R⁸ und R⁹, welche gleich oder verschieden sein können, ausgewählt sind aus H oder einem C₁₋₆-Alkylrest, wobei der C₁₋₆-Alkylrest ferner mit einem oder mehreren Substituenten substituiert sein kann, ausgewählt aus einem Halogenatom, -CN, -CF₃ und -OH;
R³ ausgewählt ist aus H, einem Halogenatom, -CN, -CF₃, einem C₁₋₄-Alkylrest oder einem C₁₋₄-Alkoxyrest;
und Salze und Solvate davon.

2. Verbindung der Formel (I) nach Anspruch 1, wobei R¹ H oder ein C₁₋₄-Alkylrest ist.

3. Verbindung der Formel (I) nach entweder Anspruch 1 oder Anspruch 2, wobei sich R² an der C(2)-Position des Pyridinylrings befindet und eine Phenylgruppe darstellt, welche ferner mit einem oder mehreren Substituenten wie in Anspruch 1 definiert substituiert sein kann.

4. Verbindung der Formel (I) nach Anspruch 3, wobei sich R² an der C(2)-Position des Pyridinylrings befindet und eine Phenylgruppe darstellt, welche ferner an der *para*-Position mit einem oder mehreren Substituenten wie in Anspruch 1 definiert substituiert sein kann.

5. Verbindung der Formel (I) nach einem der Ansprüche 1 bis 4, wobei sich R² an der C(2)-Position des Pyridinylrings befindet und eine Thiophenylgruppe darstellt, welche ferner mit einem oder mehreren Substituenten wie in Anspruch 1 definiert substituiert sein kann.

6. Verbindung der Formel (I) nach einem der vorstehenden Ansprüche, wobei sich R³ an der C(3)- oder C(6)-Position des Pyridinrings befindet und ausgewählt ist aus H, einem Halogenatom, -CN, -CF₃, einem C₁₋₄-Alkylrest oder einem C₁₋₄-Alkoxyrest.

7. Verbindung der Formel (I) nach Anspruch 1, ausgewählt aus:
2-Phenyl-4-(3-pyridin-2-yl-1H-pyrazol-4-yl)pyridin;
2-[4-(Trifluormethyl)phenyl]-4-(3-pyridin-2-yl-1H-pyrazol-4-yl)pyridin;
2-(4-Methoxyphenyl)-4-(3-pyridin-2-yl-1H-pyrazol-4-yl)pyridin;
2-(4-Fluorphenyl)-4-(3-pyridin-2-yl-1H-pyrazol-4-yl)pyridin;
2-(4-Chlorphenyl)-4-(3-pyridin-2-yl-1H-pyrazol-4-yl)pyridin;
2-(Furan-2-yl)-4-(3-pyridin-2-yl-1H-pyrazol-4-yl)pyridin;
2-(4-Trifluormethoxyphenyl)-4-(3-pyridin-2-yl-1H-pyrazol-4-yl)pyridin;
2-(4-Methylphenyl)-4-(3-pyridin-2-yl-1H-pyrazol-4-yl)pyridin;
2-(4-Ethylphenyl)-4-(3-pyridin-2-yl-1H-pyrazol-4-yl)pyridin;
2-(Thiophen-3-yl)-4-(3-pyridin-2-yl-1H-pyrazol-4-yl)pyridin;
2-[4-(1-Methyl-1H-imidazol-2-ylmethoxy)phenyl]-4-(3-pyridin-2-yl-1H-pyrazol-4-yl)pyridin;
2-[4-(3-Methyl-3H-imidazol-4-ylmethoxy)phenyl]-4-(3-pyridin-2-yl-1H-pyrazol-4-yl)pyridin;
2-[4-(2-{1H-Imidazol-1-yl}ethoxy)phenyl]-4-(3-pyridin-2-yl-1H-pyrazol-4-yl)pyridin;
2-[4-(2-Pyrrolidin-1-ylethoxy)phenyl]-4-(3-pyridin-2-yl-1H-pyrazol-4-yl)pyridin;
3-({4-[4-(3-Pyridin-2-yl-1H-pyrazol-4-yl)pyridin-2-yl]benzyl} amino)propannitril;
4-{4-[4-(3-Pyridin-2-yl-1H-pyrazol-4-yl)pyridin-2-yl]phenyl}morpholin;
2-[4-(1-Methyl-1H-imidazol-4-ylmethoxy)phenyl]-4-(3-Pyridin-2-yl-1H-pyrazol-4-yl)pyridin;
{{4-[4-(3-Pyridin-2-yl-1H-pyrazol-4-yl)pyridin-2-yl]benzyl} tetrahydropyran-4-yl)amin;
{4-[4-(3-Pyridin-2-yl-1H-pyrazol-4-yl)pyridin-2-yl]benzyl}morpholinhydrochlorid;
Pyridin-3-ylmethyl-{4-[4-(3-pyridin-2-yl-1H-pyrazol-4-yl)pyridin-2-yl]benzyl}amin;
2-Piperidin-1-yl-N-{4-[4-(3-pyridin-2-yl-1H-pyrazol-4-yl)pyridin-2-yl]phenyl}acetamid;
2-Pyrrolidin-1-yl-N- {4-[4-(3-pyridin-2-yl-1H-pyrazol-4-yl)pyridin-2-yl]phenyl}acetamid;
2-Morpholin-1-yl-N-{4-[4-(3-pyridin-2-yl-1H-pyrazol-4-yl)pyridin-2-yl]phenyl}acetamid;
2-(4-Methylpiperazin-1-yl)-N-{4-[4-(3-pyridin-2-yl-1H-pyrazol-4-yl)pyridin-2-yl]phenyl} acetamid;
3-Piperidin-1-yl-N- {4-[4-(3-pyridin-2-yl-1H-pyrazol-4-yl)pyridin-2-yl]phenyl}propionamidhydrochlorid;
3-Morpholin-4-yl-N-{4-[4-(3-pyridin-2-yl-1H-pyrazol-4-yl)pyridin-2-yl]phenyl}propionamid;
3-(4-Methylpiperazin-1-yl)-N-{4-[4-(3-pyridin-2-yl-1H-pyrazol-4-yl)pyridin-2-yl]phenyl}propionamid;
4-[4-(3-Pyridin-2-yl-1H-pyrazol-4-yl)pyridin-2-yl]-N-(tetrahydropyran-4-yl)benzamid;
N-(1-Ethylpyrrolidin-2-ylmethyl)-4-[4-(3-pyridin-2-yl-1H-pyrazol-4-yl)pyridin-2-yl]benzamid;
(1-Ethyl)-{4-[4-(3-pyridin-2-yl-1H-pyrazol-4-yl)pyridin-2-yl]phenyl}piperazin;
(N-Methyl)-({4-[4-(3-pyridin-2-yl-1H-pyrazol-4-yl)pyridin-2-yl]phenyl}tetrahydropyran-4-yl)amin;
({4-[4-(3-Pyridin-2-yl-1H-pyrazol-4-yl)pyridin-2-yl]phenyl} tetrahydropyran-4-yl)amin;
N-Methyl-({4-[4-(3-pyridin-2-yl-1H-pyrazol-4-yl)pyridin-2-yl]benzyl}tetrahydropyran-4-yl)amin;
4-Methoxy-1- {4-[4-(3-pyridin-2-yl-1H-pyrazol-4-yl)pyridin-2-yl]benzyl}piperidin;
{1-[4-(3-Pyridin-2-yl-1H-pyrazol-4-yl)pyridin-2-yl]benzyl}pyrrolidin;
(1-Methyl)-{4-[4-(3-pyridin-2-yl-1H-pyrazol-4-yl)pyridin-2-yl]benzyl}piperazin;
(1-Methylpiperidin-4-yl)-{4-[4-(3-pyridin-2-yl-1H-pyrazol-4-yl)pyridin-2-yl]benzyl}amin;
1-Methyl-4- {4-[4-(3-pyridin-2-yl-1H-pyrazol-4-yl)pyridin-2-yl]benzoyl}piperazin;
4-{4-[4-(3-Pyridin-2-yl-1H-pyrazol-4-yl)pyridin-2-yl]benzoyl}morpholin;
(4-Dimethylamino)-1-{4-[4-(3-pyridin-2-yl-1H-pyrazol-4-yl)pyridin-2-yl]benzoyl}piperidin;
(1-Methyl)-{4-[4-(3-pyridin-2-yl-1H-pyrazol-4-yl)pyridin-2-yl]phenyl}piperazin;
{4-[4-(3-Pyridin-2-yl-1H-pyrazol-4-yl)pyridin-2-yl]benzyl}thiomorpholin;
Dimethyl-{4-[4-(3-pyridin-2-yl-1H-pyrazol-4-yl)pyridin-2-yl]benzyl}amin;
4-[4-(3-Pyridin-2-yl-1H-pyrazol-4-yl)pyridin-2-yl]-N-(tetrahydropyran-4-ylmethyl)benzamid;
N-(2-Methoxyethyl)-N-methyl-4-[4-(3-pyridin-2-yl-1H-pyrazol-4-yl)pyridin-2-yl]benzarnid;
N-(2-Methoxyethyl)-4-[4-(3-pyridin-2-yl-1H-pyrazol-4-yl)pyridin-2-yl]-benzamid; und
N-Cyclohexylmethyl-4-[4-(3-pyridin-2-yl-1H-pyrazol-4-yl)pyridin-2-yl]benzamid;
und Salze und Solvate davon.

8. Arzneimittel, umfassend eine Verbindung der Formel (I) nach einem der Ansprüche 1 bis 7, zusammen mit einem pharmazeutisch verträglichen Verdünnungsmittel oder Träger.

9. Verbindung der Formel (I) nach einem der Ansprüche 1 bis 7 zur Verwendung als ein Medikament.

10. Verwendung der Verbindung der Formel (I) nach einem der Ansprüche 1 bis 7 bei der Herstellung eines Medikaments zur Behandlung oder Prophylaxe einer durch Überexpression von TGF-β gekennzeichneten Störung.

11. Verwendung einer Verbindung der Formel (I) nach Anspruch 10, wobei die Störung aus Fibrose, insbesondere Leber- und Nierenfibrose, Krebsentwicklung, abnormaler Knochenfunktion und entzündlichen Störungen und Narbenbildung ausgewählt wird.

12. Verwendung einer Verbindung der Formel (I) nach Anspruch 10 oder 11, in Kombination mit einem Antivirusmittel oder in Kombination mit ACE-Hemmern oder Angio-tensin-II-Rezeptor-Antagonisten.

13. Verfahren zur Herstellung einer Verbindung der Formel (I), 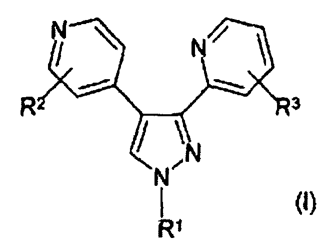 wobei
R¹ gleich H ist;
R² ausgewählt ist aus einer Phenyl-, Furanyl- oder Thiophenylgruppe, wobei die Phenylgruppe ferner mit einem oder mehreren Substituenten substituiert sein kann, welche gleich oder verschieden sein können, ausgewählt aus einem Halogenatom (wie Fluor, Chlor, Brom), -CN, -CF₃, -OCF₃, einem C₁₋₄-Alkylrest, -OR⁶, -O-(CH₂)ₙ-XR⁶R⁷, -O-(CH₂)ₙ-OR⁶, -O-(CH₂)ₙ-COR⁶, -O-(CH₂)ₙ-C₂-₆-Alkenyl, -O-(CH₂)ₙ-C₂₋₆-Alkinyl, -(CH₂)ₙ-NR⁶R⁷, -CONR⁶R⁷, -NHCOR⁶ und -NR⁶R⁷, wobei n eine ganze Zahl mit einem Wert von 1 bis 6 ist und wobei X ein Atom ist, ausgewählt aus C, N oder S, und wobei die Furanyl- und Thiophenylgruppe ferner mit einem oder mehreren Substituenten substituiert sein können, welche gleich oder verschieden sein können, ausgewählt aus einem Halogenatom (wie Fluor, Chlor, Brom), -CN, -CF₃, -OH, -OCF₃, einem C₁₋₄-Alkylrest und einem C₁₋₄-Alkoxyrest;
R⁶ und R⁷, welche gleich oder verschieden sein können, ausgewählt sind aus H, einem C₁₋₆-Alkyl-, Cycloalkyl-, Cycloalkyl-C₁₋₆-alkyl-, Aryl-, Aryl-C₁₋₆-alkyl-, Heteroaryl-, Heteroaryl-C₁₋₆-alkyl-, Heterocyclyl-, Heterocyclyl-C₁₋₆-alkyl-, C₁₋₄-Alkoxy-C₁₋₆-alkyl-, Hydroxy-C₁₋₆-alkylrest, -(CH₂)ₙ-NR⁸R⁹, oder R⁶ und R⁷ zusammen mit dem Atom, an das sie gebunden sind, einen 3-, 4-, 5-, 6- oder 7-gliedrigen gesättigten oder ungesättigten Ring bilden, welcher ein oder mehrere aus N, S oder O ausgewählte Heteroatome enthalten kann und wobei der Ring ferner mit einem oder mehreren Substituenten substituiert sein kann, ausgewählt aus einem Halogenatom (wie Fluor, Chlor, Brom), -CN, -CF₃, -OH, -OCF₃, einem C₁₋₄-Alkylrest, einem C₁₋₄-Alkoxyrest und NR⁸R⁹;
R⁸ und R⁹, welche gleich oder verschieden sein können, ausgewählt sind aus H oder einem C₁₋₆-Alkylrest, wobei der C₁₋₆-Alkylrest ferner mit einem oder mehreren Substituenten substituiert sein kann, ausgewählt aus einem Halogenatom, -CN, -CF₃ und -OH;
R³ ausgewählt ist aus H, einem Halogenatom, -CN, -CF₃, einem C₁₋₄-Alkylrest oder einem C₁₋₄-Alkoxyrest;
wobei das Verfahren umfasst:
a) Zugabe von Dimethylformamiddimethylacetal zu einem Keton der Formel (B) 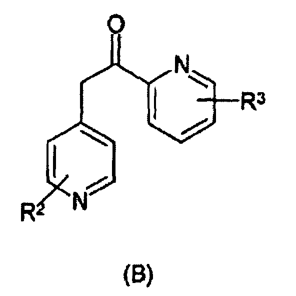 wobei R² und R³ wie vorstehend definiert sind; und
b) anschließende Zugabe von Hydrazinmonohydrat, NH₂NH₂·H₂O zu dem resultierenden Reaktionsgemisch.

14. Verfahren zur Herstellung einer Verbindung der Formel (I) 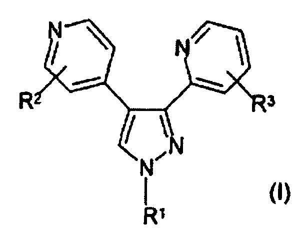 wobei
R¹ ein C₁₋₄-Alkylrest oder CH₂CONR⁴R⁵ ist, wobei R⁴ ausgewählt ist aus H oder einem C₁₋₄-Alkylrest und R⁵ ein C₁₋₄-Alkylrest ist;
R² ausgewählt ist aus einer Phenyl-, Furanyl- oder Thiophenylgruppe, wobei die Phenylgruppe ferner mit einem oder mehreren Substituenten substituiert sein kann, welche gleich oder verschieden sein können, ausgewählt aus einem Halogenatom (wie Fluor, Chlor, Brom), -CN, -CF₃, -OCF₃, einem C₁₋₄-Alkylrest, -OR⁶, -O-(CH₂)ₙ-XR⁶R⁷, -O-(CH₂)ₙ-OR⁶, -O-(CH₂)ₙ-COR⁶, -O-(CH₂)ₙ-C₂₋₆-Alkenyl, -O-(CH₂)ₙ-C₂₋₆-Alkinyl, -(CH₂)ₙ-NR⁶R⁷, -CONR⁶R⁷, -NHCOR⁶ und -NR⁶R⁷, wobei n eine ganze Zahl mit einem Wert von 1 bis 6 ist und wobei X ein Atom ist, ausgewählt aus C, N oder S, und wobei die Furanyl- und Thiophenylgruppe ferner mit einem oder mehreren Substituenten substituiert sein können, welche gleich oder verschieden sein können, ausgewählt aus einem Halogenatom (wie Fluor, Chlor, Brom), -CN, -CF₃, -OH, -OCF₃, einem C₁₋₄-Alkylrest und einem C₁₋₄-Alkoxyrest;
R⁶ und R⁷, welche gleich oder verschieden sein können, ausgewählt sind aus H, einem C₁₋₆-Alkyl-, Cycloalkyl-, Cycloalkyl-C₁₋₆-alkyl-, Aryl-, Aryl-C₁₋₆-alkyl-, Heteroaryl-, Heteroaryl-C₁₋₆-alkyl-, Heterocyclyl-, Heterocyclyl-C₁₋₆-alkyl-, C₁₋₄-Alkoxy-C₁₋₆-alkyl-, Hydroxy-C₁₋₆-alkylrest, -(CH₂)ₙ-NR⁸R⁹, oder R⁶ und R⁷, zusammen mit dem Atom, an das sie gebunden sind, einen 3-, 4-, 5-, 6- oder 7-gliedrigen gesättigten oder ungesättigten Ring bilden, welcher ein oder mehrere aus N, S oder O ausgewählte Heteroatome enthalten kann und wobei der Ring ferner mit einem oder mehreren Substituenten substituiert sein kann, ausgewählt aus einem Halogenatom (wie Fluor, Chlor, Brom), -CN, -CF₃, -OH, -OCF₃, einem C₁₋₄-Alkylrest, einem C₁₋₄-Alkoxyrest und NR⁸R⁹;
R⁸ und R⁹, welche gleich oder verschieden sein können, ausgewählt sind aus H oder einem C₁₋₆-Alkylrest, wobei der C₁₋₆-Alkylrest ferner mit einem oder mehreren Substituenten substituiert sein kann, ausgewählt aus einem Halogenatom, -CN, -CF₃ und -OH;
R³ ausgewählt ist aus H, einem Halogenatom, -CN, -CF₃, einem C₁₋₄-Alkylrest oder einem C₁₋₄-Alkoxyrest;
wobei das Verfahren die N-Alkylierung einer Verbindung der Formel (I), wobei R¹ gleich H ist, in Gegenwart einer geeigneten Base umfasst.

## Revendications

1. Composé de formule (I), 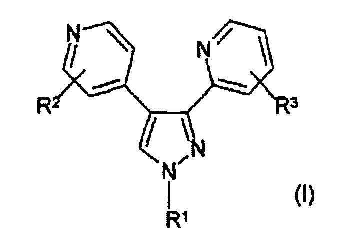 dans laquelle,
R¹ est choisi entre H, des groupes alkyle en C₁ à C₄ et CH₂CONR⁴R⁵, dans lesquels R⁴ est choisi entre H et un groupe alkyle en C₁ à C₄ et R⁵ représente un groupe alkyle en C₁ à C₄ ;
R² est choisi entre des groupes phényle, furannyle et thiophényle, dans lesquels le groupe phényle peut être en outre substitué avec un ou plusieurs substituants, qui peuvent être identiques ou différents, choisis entre des substituants halogéno (par exemple fluoro, chloro, bromo), -CN, -CF₃, -OCF₃, alkyle en C₁ à C₄, -OR⁶, -O-(CH₂)ₙ-XR⁶R⁷, -O-(CH₂)ₙ-OR⁶, -O(CH₂)ₙ-COR⁶, -O(CH₂)ₙ(alcényle en C₂ à C₆), -O-(CH₂)ₙ(alcynyle en C₂ à C₆), -(CH₂)ₙ-NR⁶R⁷, -CONR⁶R⁷, -NHCOR⁶ et -NR⁶R⁷ dans lesquels n représente un nombre entier de 1 à 6 et X représente un atome choisi entre C, N et S, et les groupes furannyle et thiophényle peuvent être en outre substitués avec un ou plusieurs substituants, qui peuvent être identiques ou différents, choisis entre des substituants halogéno (par exemple fluoro, chloro, bromo), -CN, -CF₃, -OH, -OCF₃, alkyle en C₁ à C₄ et alkoxy en C₁ à C₄ ;
R⁶ et R⁷, qui peuvent être identiques ou différents, sont choisis entre H, des groupes alkyle en C₁ à C₆, cycloalkyle, cycloalkyl (alkyle en C₁ à C₆), aryle, aryl(alkyle en C₁ à C₆), hétéroaryle, hétéroaryl(alkyle en C₁ à C₆), hétérocyclyle, hétérocyclyl (alkyle en C₁ à C₆), (alkoxy en C₁ à C₄)-(alkyle en C₁ à C₆), hydroxyalkyle en C₁ à C₆ , -(CH₂)ₙNR⁸R⁹, ou bien R⁶ et R⁷, conjointement avec l'atome auquel ils sont fixés, forment un noyau tri-, tétra-, penta-, hexa- ou heptagonal saturé ou insaturé qui peut contenir un ou plusieurs hétéroatomes choisis entre N, S et O, et le noyau pouvant être en outre substitué avec un ou plusieurs substituants choisis entre des substituants halogène (par exemple fluoro, chloro, bromo), -CN, -CF₃, -OH, -OCF₃, alkyle en C₁ à C₄, alkoxy en C₁ à C₄ et -NR⁸R⁹ ;
R⁸ et R⁹, qui peuvent être identiques ou différents, sont choisis entre H et un groupe alkyle en C₁ à C₆, le groupe alkyle en C₁ à C₆ pouvant être en outre substitué avec un ou plusieurs substituants choisis entre des substituants halogéno, -CN, -CF₃ et -OH ;
R³ est choisi entre H, des groupes halogéno, -CN, -CF₃, alkyle en C₁ à C₄ et alkoxy en C₁ à C₄ ;
et ses sels et produits de solvatation.

2. Composé de formule (I) suivant la revendication 1, dans lequel R¹ représente H ou un groupe alkyle en C₁ à C₄.

3. Composé de formule (I) suivant la revendication 1 ou la revendication 2, dans lequel R² est situé en position C(2) du noyau pyridinyle et représente un groupe phényle qui peut être en outre substitué avec un ou plusieurs substituants tels que définis dans la revendication 1.

4. Composé de formule (I) suivant la revendication 3, dans lequel R² est situé en position C(2) du noyau pyridinyle et représente un groupe phényle qui peut être en outre substitué en position *para* avec un ou plusieurs substituants tels que définis dans la revendication 1.

5. Composé de formule (I) suivant l'une quelconque des revendications 1 à 4, dans lequel R² est situé en position C(2) du noyau pyridinyle et représente un groupe thiophényle qui peut être en outre substitué avec un ou plusieurs substituants tels que définis dans la revendication 1.

6. Composé de formule (I) suivant l'une quelconque des revendications précédentes, dans lequel R³ est situé en position C(3) ou C(6) du noyau pyridine et est choisi entre H, des groupes halogéno, -CN, -CF₃, alkyle en C₁ à C₄ ou alkoxy en C₁ à C₄.

7. Composé de formule (I) suivant la revendication 1, choisi entre :
2-phényl-4-(3-pyridine-2-yl-1H-pyrazole-4-yl)pyridine ;
2- [4-(trifluorométhyl)phényl] -4- (3-pyridine-2-yl-1H-pyrazole-4-yl)pyridine ;
2-(4-méthoxyphényl)-4-(3-pyridine-2-yl-1H-pyrazole-4-yl)pyridine ;
2- (4-fluorophényl) -4- (3-pyridine-2-yl-1H-pyrazole-4-yl) pyridine ;
2-(4-chlorophényl)-4-(3-pyridine-2-yl-1H-pyrazole-4-yl)pyridine ;
2- (furanne-2-yl) -4- (3-pyridine-2-yl-1H-pyrazole-4-yl) pyridine ;
2-(4-trifluorométhoxyphényl)-4-(3-pyridine-2-yl-1H-pyrazole-4-yl)-pyridine ;
2-(4-méthylphényl)-4-(3-pyridine-2-yl-1H-pyrazole-4-yl)pyridine ;
2-(4-éthylphényl)-4-(3-pyridine-2-yl-1H-pyrazole-4-yl)pyridine ;
2-(thiophène-3-yl)-4-(3-pyridine-2-yl-1H-pyrazole-4-yl)pyridine ;
2-[4-(1-méthyl-1H-imidazole-2-ylméthoxy)-phényl]-4-(3-pyridine-2-yl-1H-pyrazole-4-yl)pyridine ;
2-[4-(3-méthyl-3H-imidazol-4-ylméthoxy)-phényl] -4- (3-pyridine-2-yl-1H-pyrazole-4-yl)pyridine ;
2-[4-(2-{1H-imidazol-1-yl}-éthoxy)-phényl]-4-(3-pyridine-2-yl-1H-pyrazole-4-yl)pyridine ;
2-[4-(2-pyrrolidine-1-yléthoxy)-phényl]-4-(3-pyridine-2-yl-1H-pyrazole-4-yl)pyridine ;
3-({4-[4-(3-pyridine-2-yl-1*H*-pyrazole-4-yl)pyridine-2-yl]benzyl}amino)propanenitrile ;
4-{4-[4-(3-pyridine-2-yl-1H-pyrazole-4-yl)-pyridine-2-yl]-phényl}morpholine ;
2- [4- (1-méthyl-1H-imidazol-4-ylméthoxy) -phényl] -4 - (3-pyridine-2-yl-1H-pyrrazole-4-yl)pyridine ;
({4-[4-(3-pyridine-2-yl-1H-pyrazole-4-yl)pyridine-2-yl]benzyl}tétrahydropyranne-4-yl)-amine ;
chlorhydrate de {4-[4-(3-pyridine-2-yl-1H-pyrazole-4-yl)pyridine-2-yl]benzyl}-morpholine ;
pyridine-3-ylméthyl-{4-[4-(3-pyridine-2-yl-1H-pyrazole-4-yl)pyridine-2-yl]benzyl}-amine ;
2-pipéridine-1-yl-N-{4-[4-(3-pyridine-2-yl-1H-pyrazole-4-yl)-pyridine-2-yl]-phényl}-acétamide ;
2-pyrrolidine-1-yl-N-{4-[4-(3-pyridine-2-yl-1H-pyrazole-4-yl)-pyridine-2-yl]-phényl}-acétamide ;
2-morpholine-1-yl-N-{4- [4- (3-pyridine-2-yl-1H-pyrazole-4-yl)-pyridine-2-yl]-phényl}-acétamide ;
2 - (4-méthylpipérazine-1-yl) -N-{4- [4 - (3-pyridine-2-yl-1H-pyrazole-4-yl)-pyridine-2-yl]-phényl}-acétamide ;
chlorhydrate de 3-pipéridine-1-yl-N-{4-[4-(3-pyridine-2-yl-1H-pyrazole-4-yl)-pyridine-2-yl]-phényl}-propionamide ;
3-morpholine-4-yl-N-{4-[4-(3-pyridine-2-yl-1H-pyrazole -4-yl) - pyridine-2-yl] -phényl}-propionamide ;
3- (4-méthylpipérazine-1-yl) -N-{4- [4- (3-pyridine-2-yl-1H-pyrazole-4-yl)-pyridine-2-yl]-phényl}-propionamide ;
4-[4-(3-pyridine-2-yl-1H-pyrazole-4-yl)-pyridine-2-yl]-N-(tétrahydro-pyranne-4-yl)-benzamide ;
N- (1 - éthylpyrrolidine - 2 -ylméthyl) 4 - [4 - (3 -pyridine - 2 -yl-1H-pyrazole-4-yl)-pyridine-2-yl]-benzamide ;
(1-éthyl) -{4- [4-(3-pyridine-2-yl-1*H*-pyrazole-4-yl)pyridine-2-yl]-phényl}-pipérazine ;
(*N*-méthyl)-({4-[4-(3-pyridine-2-yl-1*H*-pyrazole-4-yl)pyridine-2-yl]-phényl}-1-tétrahydropyranne-4-yl)-amine ;
({4- [4- (3-pyridine-2-yl-1H pyrazole-4-yl) pyridine-2 -yl ]-phényl}-tétrahydropyranne-4-yl)-amine ;
*N*-méthyl-({4- [4- (3-pyridine-2-yl-1*H*-pyrazole-4-yl)pyridine-2-yl]benzyl}-tétrahydropyranne-4-yl)-amine ;
4-méthoxy-1-{4- [4- (3-pyridine-2-yl-1*H*-pyrazole-4-yl) pyridine-2-yl]benzyl}-pipéridine ;
{1- [4- (3-pyridine-2-yl-1*H*-pyrazole-4-yl)pyridine-2-yl]benzyl}-pyrrolidine ;
(1-méthyl)-{4-[4-(3-pyridine-2-yl-1*H*-pyrazole-4-yl)pyridine-2-yl]benzyl}-pipérazine ;
(1-méthylpipéridine-4-yl) -{4- [4- (3-pyridine-2-yl-1*H*-pyrazole-4-yl)pyridine-2-yl]benzyl}-amine ;
1-méthyl-4-{4-[4-(3-pyridine-2-yl-1*H*-pyrazole-4-yl)-pyridine-2-yl]-benzoyl}-pipérazine ;
4-{4-[4-(3-pyridine-2-yl-1*H*-pyrazole-4-yl) -pyridine-2-yl] -benzoyl}-morpholine ;
(4-diméthylamino)-1-{4-[4-(3-pyridine-2-yl-1*H*-pyrazole-4-yl)-pyridine-2-yl]-benzoyl}-pipéridine ;
(1-méthyl)-{4-[4-(3-pyridine-2-yl-1*H*-pyrazole-4-yl)pyridine-2-yl]-phényl}-pipérazine ;
{4- [4- (3-pyridine-2-yl-1H-pyrazole-4-yl) pyridine-2-yl] benzyl} -thiomorpholine ;
diméthyl-{4-[4-(3-pyridine-2-yl-1*H*-pyrazole-4-yl) pyridine-2-yl]-benzyl}-amine ;
4-[4-(3-pyridine-2-yl-1H-pyrazole-4-yl) -pyridine-2-yl] -N-(tétra-hydropyranne-4-ylméthyl)-benzamide ;
N-(2-méthoxyéthyl)-*N*-méthyl-4-[4-(3-pyridine-2-yl-1H-pyrazole-4-yl)-pyridine-2-yl]-benzamide ;
N-(2-méthoxyéthyl)-4-[4-(3-pyridine-2-yl-1H-pyrazole-4-yl)-pyridine-2-yl]-benzamide ; et
N-cyclohexylméthyl-4-[4-(3-pyridine-2-yl-1H-pyrazole-4-yl)-pyridine-2-yl]-benzamide ;
et leurs sels et produits de solvatation.

8. Composé pharmaceutique comprenant un composé de formule (I) suivant l'une quelconque des revendications 1 à 7, conjointement avec un diluant ou support pharmaceutiquement acceptable.

9. Composé de formule (I) suivant l'une quelconque des revendications 1 à 7, destiné à être utilisé comme médicament.

10. Utilisation d'un composé de formule (I) suivant l'une quelconque des revendications 1 à 7, dans la production d'un médicament destiné au traitement ou à la prophylaxie d'une affection **caractérisée par** surexpression de TGF-β.

11. Utilisation d'un composé de formule (I) suivant la revendication 10, dans laquelle l'affection est choisie entre la fibrose, notamment la fibrose hépatique et la fibrose rénale, le développement d'un cancer, une fonction osseuse anormale et des affections inflammatoires et la cicatrisation.

12. Utilisation d'un composé de formule (I) suivant la revendication 10 ou la revendication 11 en association avec un agent antiviral, ou en association avec des inhibiteurs de ACE ou des antagonistes du récepteur d'angiotensine II.

13. Procédé pour la préparation d'un composé de formule (I) : 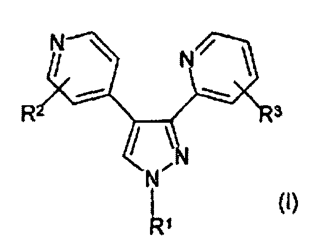 dans laquelle,
R¹ représente H ;
R² est choisi entre des groupes phényle, furannyle et thiophényle, dans lesquels le groupe phényle peut être en outre substitué avec un ou plusieurs substituants, qui peuvent être identiques ou différents, choisis entre des substituants halogéno (par exemple fluoro, chloro, bromo), -CN, -CF₃, -OCF₃, alkyle en C₁ à C₄, -OR⁶, -O-(CH₂)ₙ-XR⁶R⁷, -O- (CH₂)ₙ-OR⁶, -O(CH₂)ₙ-COR⁶, -O(CH₂)ₙ(alcényle en C₂ à C₆) , -O-(CH₂)ₙ(alcynyle en C₂ à C₆), -(CH₂)ₙNR⁶R⁷, -CONR⁶R⁷, -NHCOR⁶ et -NR⁶R⁷ dans lesquels n représente un nombre entier de 1 à 6 et X représente un atome choisi entre C, N et S, et les groupes furannyle et thiophényle peuvent être en outre substitués avec un ou plusieurs substituants, qui peuvent être identiques ou différents, choisis entre des substituants halogéno (par exemple fluoro, chloro, bromo), -CN, -CF₃, -OH, -OCF₃, alkyle en C₁ à C₄ et alkoxy en C₁ à C₄ ;
R⁶ et R⁷, qui peuvent être identiques ou différents, sont choisis entre H, des groupes alkyle en C₁ à C₆, cycloalkyle, cycloalkyl (alkyle en C₁ à C₆), aryle, aryl(alkyle en C₁ à C₆), hétéroaryle, hétéroaryl(alkyle en C₁ à C₆), hétérocyclyle, hétérocyclyl (alkyle en C₁ à C₆), (alkoxy en C₁ à C₄)-(alkyle en C₁ à C₆), hydroxyalkyle en C₁ à C₆ , -(CH₂)ₙ-NR⁸R⁹, ou bien R⁶ et R⁷, conjointement avec l'atome auquel ils sont fixés, forment un noyau tri-, tétra-, penta-, hexa- ou heptagonal saturé ou insaturé qui peut contenir un ou plusieurs hétéroatomes choisis entre N, S et O, et le noyau pouvant être en outre substitué avec un ou plusieurs substituants choisis entre des substituants halogéno (par exemple fluoro, chloro, bromo), -CN, -CF₃, -OH, -OCF₃, alkyle en C₁ à C₄, alkoxy en C₁ à C₄ et -NR⁸R⁹ ;
R⁸ et R⁹, qui peuvent être identiques ou différents, sont choisis entre H et un groupe alkyle en C₁ à C₆, le groupe alkyle en C₁ à C₆ pouvant être en outre substitué avec un ou plusieurs substituants choisis entre des substituants halogéno, -CN, -CF₃ et -OH ;
R³ est choisi entre H, des groupes halogène, -CN, -CF₃, alkyle en C₁ à C₄ et alkoxy en C₁ à C₄ ;
procédé qui comprend :
a) l'addition d'acétal diméthylique de diméthylformamide à une cétone de formule (B) 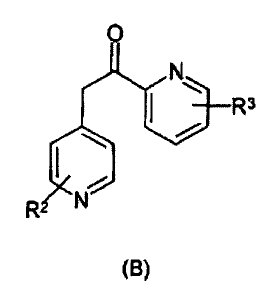 dans laquelle R² et R³ répondent aux définitions précitées ; et
b) ensuite l'addition de monohydrate d'hydrazine, NH₂NH₂.H₂O, au mélange réactionnel résultant.

14. Procédé pour la préparation d'un composé de formule (I), 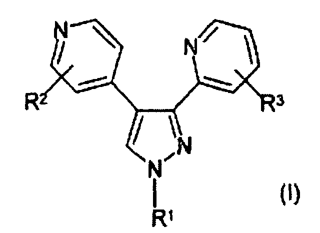 dans laquelle,
R¹ représente un groupe alkyle en C₁ à C₄ ou CH₂CONR⁴R⁵, dans lequel R⁴ est choisi entre H, un groupe alkyle en C₁ à C₄ et R⁵ représente un groupe alkyle en C₁ à C₄ ;
R² est choisi entre des groupes phényle, furannyle et thiophényle, dans lesquels le groupe phényle peut être en outre substitué avec un ou plusieurs substituants, qui peuvent être identiques ou différents, choisis entre des substituants halogéno (par exemple fluoro, chloro, bromo), -CN, -CF₃, -OCF₃, alkyle en C₁ à C₄, -OR⁶, -O-(CH₂)ₙ-XR⁶R⁷, -O-(CH₂)ₙ-OR⁶, -O(CH₂)ₙ-COR⁶, -O(CH₂)ₙ(alcényle en C₂ à C₆), -O-(CH₂)ₙ(alcynyle en C₂ à C₆) , -(CH₂)ₙ-NR⁶R⁷, -CONR⁶R⁷, -NHCOR⁶ et -NR⁶R⁷, dans lesquels n représente un nombre entier de 1 à 6 et X représente un atome choisi entre C, N et S, et les groupes furannyle et thiophényle peuvent être en outre substitués avec un ou plusieurs substituants, qui peuvent être identiques ou différents, choisis entre des substituants halogéno (par exemple fluoro, chloro, bromo), -CN, -CF₃, -OH, -OCF₃, alkyle en C₁ à C₄ et alkoxy en C₁ à C₄ ;
R⁶ et R⁷, qui peuvent être identiques ou différents, sont choisis entre H, des groupes alkyle en C₁ à C₆, cycloalkyle, cycloalkyl(alkyle en C₁ à C₆), aryle, aryl(alkyle en C₁ à C₆), hétéroaryle, hétéroaryl(alkyle en C₁ à C₆), hétérocyclyle, hétérocyclyl (alkyle en C₁ à C₆), (alkoxy en C₁ à C₄)-(alkyle en C₁ à C₆), hydroxyalkyle en C₁ à C₆ , -(CH₂)ₙ-NR⁸R⁹, ou bien R⁶ et R⁷, conjointement avec l'atome auquel ils sont fixés, forment un noyau tri-, tétra-, penta-, hexa- ou heptagonal saturé ou insaturé qui peut contenir un ou plusieurs hétéroatomes choisis entre N, S et O, et le noyau pouvant être en outre substitué avec un ou plusieurs substituants choisis entre des substituants halogéno (par exemple fluoro, chloro, bromo), -CN, -CF₃, -OH, -OCF₃, alkyle en C₁ à C₄, alkoxy en C₁ à C₄ et -NR⁸R⁹ ;
R⁸ et R⁹, qui peuvent être identiques ou différents, sont choisis entre H et un groupe alkyle en C₁ à C₆, le groupe alkyle en C₁ à C₆ pouvant être en outre substitué avec un ou plusieurs substituants choisis entre des substituants halogéno, -CN, -CF₃ et -OH ;
R³ est choisi entre H, des groupes halogéno, -CN, -CF₃, alkyle en C₁ à C₄ et alkoxy en C₁ à C₄ ;
procédé qui comprend la N-alkylation d'un composé de formule (I) dans laquelle R¹ représente H, en présence d'une base convenable.
